# EUROPEAN PATENT SPECIFICATION

(11) **EP 4 420 719 B1**
(45) Date of publication and mention of the grant of the patent: **28.05.2025**
(21) Application number: 23158167.9
(22) Date of filing: 23.02.2023
(51) Int. Cl.: A61N 5/10, G21K 1/04, G21K 1/10

(54) **DYNAMIC RIDGE FILTER AND RANGE SHIFTER AND RADIATION SYSTEM COMPRISING SAME**
DYNAMISCHER STEGFILTER UND BEREICHSSCHIEBER UND BESTRAHLUNGSSYSTEM MIT SELBIGEN
FILTRE À CRÊTE DYNAMIQUE ET DISPOSITIF DE DÉCALAGE DE PLAGE ET SYSTÈME DE RAYONNEMENT LE COMPRENANT

(43) Date of publication of application: 28.08.2024
(73) Proprietor: Ion Beam Applications, 1348 Louvain-la-Neuve (BE)
(72) Inventor: CLAEREBOUDT, Yves, 1348 Louvain-la-Neuve (BE)
(74) Representative: Connor, Marco Tom

(56) References cited:
- WO-A1-2014/132481
- US-A1- 2006 226 372
- US-A1- 2011 240 874

## Description

### TECHNICAL FIELD

The present invention concerns a dynamic shaping device for shaping the Spread-Out Bragg Peaks (= SOBP) of pencil beams, that can be adjusted to adapt to the geometry of any target volume comprising tumoural cells or other conditions requiring irradiation. The shaping device comprises a dynamic ridge filter and / or a dynamic range shifter. The dynamic shaping device of the present invention comprises a number of identical rows formed by a number of modules of different predefined geometries. Each row is configured to translate independently along a Y-axis to define a string formed by a selection of one module of each row aligned along an X-axis (X ⊥ Y) and configured to shape the dose deposited onto the specific volume facing the string.

The dynamic shaping device of the present invention has the advantage that a single dynamic shaping device can be used to shape the pencil beams for treating different tumours of different geometries. It allows substantial time and cost saving as it is not necessary to produce a new shaping device for every patient and, also, for different irradiation sessions to a same patient, as the geometry of a tumour and patient can evolve with time. Having a dynamic shaping device allows for immediate treatment plan adaptation.

### BACKGROUND OF THE INVENTION

Radiation therapy with particles or waves, such as electron beams, protons beams, heavy ions beams, x-rays, γ-rays, and the like, has become an essential tool for treating patients with tumours.

Since both tumoural cells and healthy cells comprised in a volume are irradiated, a first challenge in cancer treatment is to define a treatment plan ensuring that defined doses are deposited into the tumoural cells to effectively destroy or kill them, while limiting the doses deposition into healthy cells to spare them as much as possible. A second challenge is to actually deposit the defined doses onto the tumoural cells whilst limited doses are actually deposited onto the healthy cells as planned.

Different radiation modalities deposit their energies in different patterns. For example, X-rays deposit most of their energy near the level of the skin, and the deposited energy decreases with penetration depth into the tissues. Healthy tissues located upstream of a treatment volume of tumoural cells therefore receive a higher dose than the cells located in the treatment volume. By contrast, charged particle beams, such as protons and carbon ions, deposit most of their energy close to the end of their beam path, forming a so-called Bragg peak.

The charged particles emerging from a nozzle of a particle accelerator form a narrow pencil beam. To cover a treatment volume of practical size, the pencil beam must be spread laterally either by introducing scattering material in the beam path or by scanning the beam over the desired area. Pencil beam scanning (PBS) and double scattering (DS) proton therapy are two techniques allowing physicians to deliver a precise, powerful dose of radiation that covers the tumour with minimal radiation exposure to healthy tissues.

PBS is very advantageous because it optimizes the geometrical distribution of the dose deposition to match it with the geometry of the treatment volume enclosing the tumour. PBS treatment time can, however, be long as the PBS-beams must scan over each spot and over multiple mono-energetic layers. Moving a PBS-beam from onemono-energectic layer to the next one with different energies is time consuming, of the order of 500 ms. It is therefore advantageous, when irradiation time is a factor, such as in FLASH-RT, to reduce the number of layers, optimally to a single layer.

Saving treatment time reduces the operation time of a particle accelerator by each patient. It is also more comfortable for the patient. It is also advantageous if the treatment plan comprises FLASH irradiation, wherein doses are deposited into the cells at high dose rates (HDR), of at least 1 Gy / s or even up to at least 40 Gy / s. A given high dose (e.g., 5 to 10 Gy) deposited at HDR has shown to better spare healthy cells relative to the same dose deposited at lower conventional dose rates (CDR). Where FLASH irradiation is particularly interesting, is that a given dose deposited into tumoural cells has the same killing effect irrespective of whether it was deposited at HDR or at CDR.

"Painting" a target volume in depth with a single layer can be achieved by shaping the pencil beams delivered towards the volume to be treated by means of a shaping device intersecting the pencil beam path. A shaping device is a device positioned between a source of accelerated particles and a treatment volume, changing the energy profile and / or the geometry of the beam traversing it. It is designed for absorbing a portion of the required energy of a portion of the protons from the pencil beams to control the specific volumes where doses are deposited onto by each pencil beam. The present invention concerns more particularly ridge filters and range shifters and shaping devices composed of at least one ridge filter and at least one range shifter.

A CT-scan image of a patient is produced, which values are converted to proton stopping power. A treatment plan is established by a practitioner defining the doses to be deposited onto specific volumes. One or more shaping devices are designed by a treatment planning system (TPS) which shapes the beam to match a geometry of the treatment volume comprising tumoral cells, for depositing specific doses onto specific locations within the treatment volume according to the treatment plan while minimizing the dose deposited onto healthy tissues

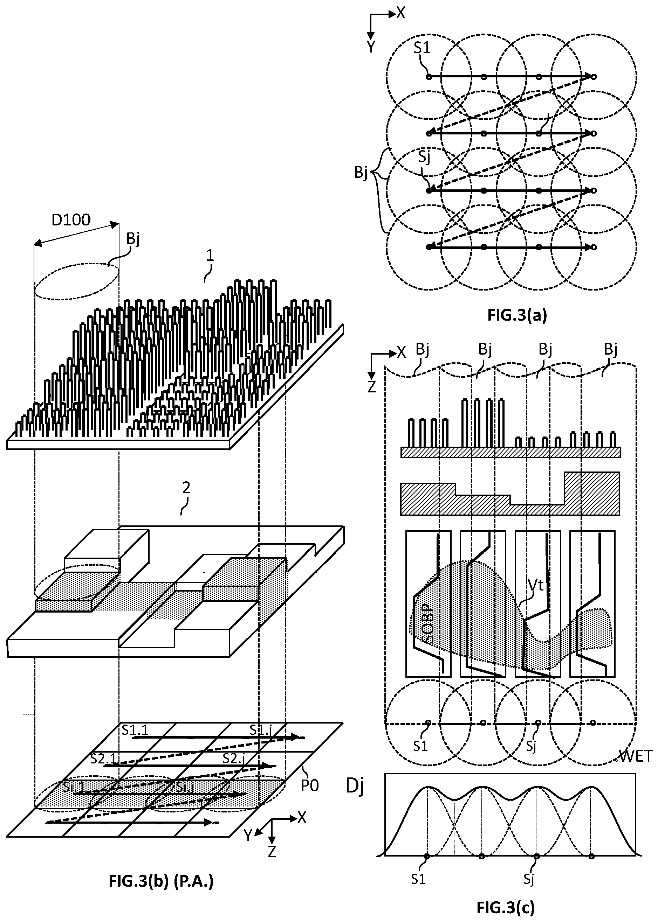

A ridge filter allows shaping the Spread-Out Bragg Peak (SOBP) along the corresponding pencil beam axis (Zj). As illustrated in Figures 3(b) and 3(c). Ridge filters are known in the art and comprise energy spreading units distributed on a base. They are in the form of smooth-pins or step pyramids, or crests extending along the beam axes (Zj) of the individual pencil beams. For example, EP21208699 describes a ridge filter comprising a plurality of energy spreading units in the form of orifices or pins arranged side by- side according to the- array of spots in a support base. Each energy spreading unit is formed by one or more spreading subunits in the form of orifices or pins having a generalized cylindrical geometry of cross-sectional areas and extending along the corresponding beam axis (Zj) from the- support block. The spreading subunits can for example be stacked on top of one another along the corresponding irradiation axis (Zj). The superposition of spreading subunits forming each energy spreading unit allows shaping and varying the width of the Spread-Out Bragg Peak (SOBP) along the corresponding beam axis (Zj).

A range shifter consists of slabs of stopping material inserted between the nozzle and the treatment volume and is used to reduce the residual range of the incident beam so that the treatment ranges can be extended to shallow depths. In particular, a range shifter allows limiting the region where doses are deposited at a downstream (or distal) end of the treatment volume, relative to the particles' propagation direction. For a material of given particle stopping capacity, varying the thickness of the slabs facing each pencil beams allows match the boundary where doses are deposited to the geometry of the downstream surface of the target volume.

When used for HDR irradiation using a single mono-energetic layer, ridge filters and range shifters are unique devices usable once only for one patient and are tailor-made according to the corresponding planned device design to match the type and geometry of tumour to be treated. They can be produced by machining, but they are generally produced by 3D-printing, which is still time-consuming and relatively expensive (although less than by machining). The shaping devices could also need replacement by new ones for treatment of a same tumour of a same patient in different irradiation sessions because, as shown in CT-scan images, the geometry of the tumour or patient varies with time and with the preceding irradiation sessions leading to a dose distribution unacceptable by clinicians if the shaping devices are unchanged. A particle beam irradiation apparatus and therapy system with a dynamic range shifter are known from US 2011240874 A1.

The present invention proposes a dynamic shaping device, including a dynamic ridge filter and / or a dynamic range shifter, that can be re-used several times and adapted for the treatment of tumours of different geometries. These and other advantages of the present invention are described in continuation.

### SUMMARY OF THE INVENTION

The present invention is defined in the appended independent claims. Preferred embodiments are defined in the dependent claims. In particular, the present invention concerns a dynamic ridge filter, a dynamic range shifter, and a dynamic shaping device comprising the dynamic ridge filter and the dynamic range shifter, each of them being configured for shaping a dose deposition zone by radiation with charged particles beams, preferably with proton or other light ion beams, and for depositing doses (Dj) applied by pencil beams (Bj) propagating along corresponding irradiation axes (Zj) fanning out of a central irradiation axis (Z) by a pencil beam scanning (PBS) system and delivered in sequence to specific volumes (Vj) along a X-axis to form specific volume stripes distributed next to one another along a Y-axis transverse, preferably normal, to the X-axis, the specific volumes (Vj) defining in combination a target volume (Vt) comprising the tumoral cells,

The dynamic ridge filter comprises a number of energy spreading units, each energy spreading unit being characterized by a corresponding energy spreading capacity for spreading the dose deposited along the irradiation axis (Zj) by the corresponding pencil beams (Bj) traversing one or more energy spreading units. The gist of the dynamic ridge filter is that the energy spreading units are distributed in filter modules, each filter module supporting one or more energy spreading units and having an area normal to the central irradiation axis (Z) compatible with a cross-section of the corresponding pencil beam (Bj). The filter modules are arranged in different filter rows, each extending along the row-direction (Y).

Each filter row comprises a number of dissimilar filter modules. Each filter module of a filter row has a different energy spreading capacity from the other filter modules of the same filter row. Each filter row can be displaced independently to form a filter string along the X-axis and the filter string is configured to shape the dose deposition in the specific volumes (vj) forming the specific volume stripe facing the filter string along the irradiation axes (Zj). All filter rows are preferably identical and comprise a same selection of filter modules, to be combined to form a corresponding filter string.

In an embodiment, the energy spreading units are in the form of spikes of generalized cylindrical, preferably prismatic geometry, or of conical geometry, truncated or not, preferably a pyramidal geometry. The spikes are supported on a base of the filter modules and are configured in use for having a major axis of the spikes extending parallel to the corresponding pencil beam axes (Zj).

In an alternative embodiment, the energy spreading units are in the form of orifices of generalized cylindrical or conical geometry penetrating in a support base of the filter modules. Each orifice extends from an aperture opening at a surface of the support base and penetrating to a given depth leaving a resulting thickness of material of the support base. The orifices are configured in use for having a major axis of the cavities extending parallel to the corresponding pencil beam axes (Zj).

Each filter module can comprise one or more energy spreading units ,each formed by a plurality of spreading subunits having differing cross-sectional areas (Ai) normal to the irradiation axis (Zj). They can be stacked on top of one another along a major axis of the energy spreading unit configured in use for extending parallel to the irradiation axis (Zj), wherein all spreading subunits of the stack have different energy spreading capacities.

The dynamic range shifter has a thickness of material varying over an area of the range shifter, the area being configured for being substantially normal to the irradiation axis (Zj) in use, wherein a value of the thickness determines a corresponding amount of absorbed energy (ΔE) of the pencil beam (Bj) of the charged particles traversing the thickness. A topography of the range shifter approximates a topography of a downstream portion of the target volume (Vt), wherein "downstream" is defined relative to the particle irradiation direction.

The gist of the range shifter of the present invention is that the range shifter comprises shifter modules arranged side-by-side to form shifter rows, wherein each shifter row comprises a selection of shifter modules having a constant or almost constant thickness different from the other shifter modules of the same shifter row, and configured for absorbing different amounts of reference absorbed energy (ΔE) from the pencil beam (Bj). The shifter row has a step-geometry. Alternatively, the shifter row can have a continuous geometry, forming a continuous slope from a high thickness to a low thickness, wherein each shifter module has a varying thickness, whose high and low values are equal to the low and high values of the thicknesses of the shifter modules adjacent thereto on either side. This geometry can be defined as an infinite number of infinitely thin slices of decreasing height measured along the central irradiation axis (Z) aligned side by side along the Y-axis.

The range shifter, with step- or continuous-geometry is such that,
- each shifter row can be displaced independently to form a shifter string along the X-axis,
- the shifter string is configured to shape along the irradiation axes (Zj) the dose deposition in the specific volumes (Vj) forming the specific volume stripe facing the shifter string along the irradiation axes (Zj).

All shifter rows are preferably identical comprising a same selection of shifter modules.

The dynamic shaping device comprises at least a dynamic ridge filter as defined supra and at least a dynamic range shifter as defined supra disposed in a sequence along a beam path of a pencil beam (Bj), such that the filter string and the shifter string face each other along a direction, which in use is parallel the irradiation axis (Zj), to form in combination a radiation string.

The present invention also concerns a treatment station comprising,
- a source of pencil beams (Bj) of accelerated charged particles, preferably of protons,
- a nozzle for directing the pencil beams (Bj) of accelerated charged particles towards the target volume (Vt), the nozzle comprising electromagnetic elements configured for deviating the pencil beam (Bj) to scan along at least the X-axis as the nozzle remains static,
- a dynamic ridge filter a defined supra
- a dynamic range shifter as defined supra,
- a couch or chair for receiving the patient in supine, prone, seated or standing position,
- one or more processors configured for controlling various components of the treatment station,
wherein the dynamic ridge filter (1) and dynamic range shifter are arranged such as to form a shaping device as defined supra.

In a preferred embodiment of the treatment station of the present invention,
- the electromagnetic elements are configured for deviating the pencil beam (Bj) to scan also along the Y-axis as the nozzle remains static, and
   ∘ the shaping device is provided with a translation system configured for following a translation of the pencil beam along the Y-axis such that the filter string and the shifter string move together with the shaping device keeping in alignment with the pencil beam along the Y-axis, or
   ∘ the filter string and the shifter string move along the Y-axis relative to the shaping device which preferably remains static relative to the target volume (Vt), in order to keep in alignment with the pencil beam along the Y-axis, or
- The couch is provided with a translation system configured for translating the couch along the Y-axis to align the specific volumes (Vj) with the corresponding pencil beams (Bj), whilst the shaping device and the filter string and shifter string preferably remain static relative to the target volume, or.
- a combination of the foregoing options.

the one or more processors are preferably configured for:
- moving the filter rows and the shifter rows along the Y-axis to yield a sequence of filter modules forming an i^{th} filter string and of an i^{th} shifter string extending along the X-axis to yield an i^{th} radiation string according to a predefined treatment plan, such that the beam paths of the pencil beams (Bj) of an i^{th} scanning string traverse corresponding radiation modules of the sequence of radiation modules forming the i^{th} radiation string (32i) before attaining the target volume (Vt), wherein a radiation module is formed by a filter module and a shifter module aligned along the irradiation axis (Z),
- controlling the electromagnetic elements (4) for orienting the pencil beam (Bj) through a first radiation module formed by a first filter module (12) of the i^{th} filter string and a first shifter module of the i^{th} shifter string, and towards a first spot (Sj) of an i^{th} scanning string formed by a sequence of spots (Sj) distributed along the X-axis, the spots being arranged in a plurality of scanning strings (i, i+1...) distributed along the Y-axis to define an array of spots arranged on a plane (X, Y) representative of a projection onto the plane (X, Y) of the treatment volume (Vt),
- after the pencil beam (Bj) traversing the first spot (Sj) of the i^{th} scanning string delivered a predefined dose (Dj), the electromagnetic elements are controlled for sequentially orienting the pencil beams (Bj) of the i^{th} scanning string through the sequence of radiation modules forming the the i^{th} radiation string (32i), and towards the sequence of spots (Sj) of the i^{th} scanning string.,
- for all filter modules forming the i^{th} filter string and for all shifter modules forming the i^{th} shifter string, after the pencil beam (Bj) has traversed an x^{th} radiation module and moves to a next (x+1)^{th} radiation module along the X-axis, moving along the Y-axis the j^{th} filter row and the x^{th} shifter row to yield the radiation module (32) required for irradiating the x^{th} radiation module of the next (i + 1)^{th} radiation string required for irradiating a next (i+1)^{th} scanning string of spots (Sj) according to the predefined treatment.

In an embodiment of the present invention, after the pencil beam (Bj) traversing a last spot (Sj) of the i^{th} scanning string delivered a predefined dose (Dj) into a corresponding last specific volume (Vj), the one or more processors are configured for,
- controlling the electromagnetic elements or the translation system of the couch as defined supra for orienting the pencil beam (Bj) through a first spot of an (i+ 1)^{th} scanning string adjacent along the Y-axis to the i^{th} scanning string, and
- repeating the foregoing steps for the spots on the (i+ 1)^{th} scanning string,
- repeating the foregoing steps for all of the plurality of scanning strings forming the array of spots (Sj) which have not yet received the dose according to the predefined treatment plan.

In an embodiment, the couch is static and, to ensure that the beam paths followed by the pencil beams (Bj) always cross a corresponding filter module of the filter string and a corresponding shifter module of the shifter string, the one or more processors are configured for synchronizing the electromagnetic elements and
- the translation system of the shaping device for ensuring that as the pencil beam is deviated along the Y-axis, the shaping device or the radiation string is also translated along the Y-axis, or
- a moving of the filter string and the shifter string along the Y-axis relative to the shaping device which remains static relative to the target volume (Vt), in order to keep in alignment with the pencil beam along the Y-axis.

### BRIEF DESCRIPTION OF THE FIGURES

For a fuller understanding of the nature of the present invention, reference is made to the following detailed description taken in conjunction with the accompanying drawings in which:
**Figure 1(a)** shows pencil beams irradiating a target volume after passing through a range shifter and a ridge filter to define SOBP's matching the dose deposition pattern onto the target volume as defined in a treatment plan.
**Figure 1(b)** shows a spot pattern on plane P0, defining the positions of the different pencil beams.
**Figures 1(c)****&1(d)** show two examples of SOBP's defining the dose within two specific volumes as desposited by pencil beams Bi and Bm, respectively, as defined in Figure 1(a).
**Figure 2(a)** shows a simplified view of the depth dose deposition around a typical Bragg peak of a mono-energetic beam of given energy. The abscissa axis represents the depth in water. The maximum beam range (W0) in water is defined as the depth beyond the maximum of the Bragg peak and corresponding to an energy equal to 80% of the maximum of the Bragg peak, wherein WO must be at least equal to or larger than dj (dj < WO).
**Figure 2(b)** shows the depth dose profile of the Bragg peak of the beam of Figure 2(a) with an energy shifting unit intersecting the path of the beam.
**Figures 2(c) to 2(e)** show the effect of one or more spreading units on the construction of a SOBP of desired shape.
**Figure 3(a)** shows an example of sequence of spots irradiated by successive pencil beams.
**Figure 3(b)** shows an example of monolithic manufactured ridge filter and range shifter of the prior art interposed between a source of irradiation and a target volume.
**Figure 3(c)** schematically shows the SOBP's obtained by pencil beams successively irradiating a series of spots aligned along the X-axis, with the corresponding dose deposition distributions formed by intersecting Gaussian distribution curves.
**Figures 4(a)****&4(b)** show perspective views of a single row of a dynamic ridge filter and of a dynamic range shifter, respectively, according to the present invention, each formed by a succession of modules of standard dimensions aligned along the Y-axis.
**Figure 4(c)** shows side views of the single rows of a dynamic ridge filter and of a dynamic range shifter of Figures 4(a) and 4(b) forming in combination of dynamic shaping device.
**Figures 5(a)****&5(b)** show an irradiation treatment station comprising a dynamic shaping device according to the present invention.
**Figures 6(a)****&6(b)** show the example of Figures 3(b)&3(c) with the monolithic ridge filter and range shifter of the prior art replaced by a dynamic ridge filter and range shifter according to the present invention. The resulting SOBP's illustrated in Figures 3(c) and 6(b) are identical.
**Figures 7(a)****&7(b)** show the required sequence of energy spreading units forming sequential filter strings for treating a given target volume, wherein the codes A, B... are defined in Figure 7(b).
**Figure 7(c)** shows the required sequence of shifter modules forming sequential shifter strings for treating a given target volume, wherein the codes a, b... are defined in Figure 7(d) & 7(e).
**Figure 7(d) & 7(e)** show two embodiments of range shifters defining the codes a, b... indicated in Figure 7(c). The range shifter of Figure 7(d) is formed of discrete modules of constant thickness, whereas the range shifter of Figure 7(e) has a continuous slope, with the boundaries of the modules a, b,... being defined by the diameter of the pencil beam.
**Figures 7(f)****&7(g)** show the required sequence of energy spreading units and shifter modules of the dynamic range shifter forming sequential radiation strings for treating a given target volume with a combination [Ba] to be applied to the spot (i, j) in Figure 7(f), wherein the codes Aa, Bb... are defined in Figure 7(f).
**Figures 8(a) to 8(d)** show the translation of rows as the pencil beams successively pass from one spot to the next one along a first scanning string parallel to the X-axis.
**Figures 9(a) to 9(d)** show the translation of rows as the pencil beams successively pass from one spot to the next one along a second scanning string parallel to the X-axis.
**Figure 10(a) to 10(e)** show different embodiments of ridge filter modules.
**Figures 11(a) to 11d)** show different embodiments of ridge filter modules, with energy spreading units formed by a superposition of various energy spreading subunits.
**Figure 12(a) to 12(c)** show different spots irradiation sequences along both X-axis and Y-axes.
**Figures 13(a) & 13(b)** schematically show an irradiation station wherein the nozzle is configured for scanning the pencil beams along both X- and Y-axes, and wherein the shaping device is configured to translate along the Y-axes to maintain the scanning string aligned with the pencil beams as they shift along the Y-axis.
**Figures 14(a) & 14(b)** schematically show an irradiation station wherein the nozzle is configured for scanning the pencil beams along both X- and Y-axes, and wherein the shaping device is configured for translating the radiation string along the Y-axes to maintain it aligned with the pencil beams as they shift along the Y-axis.
**Figures 15(a) & 15(b)** schematically show an irradiation station wherein the nozzle is configured for scanning the pencil beams along the X-axis, and wherein the couch is configured to translate along the Y-axis to allow the target volume to be scanned along the Y-axis by the pencil beams.

### DETAILED DESCRIPTION OF THE INVENTION

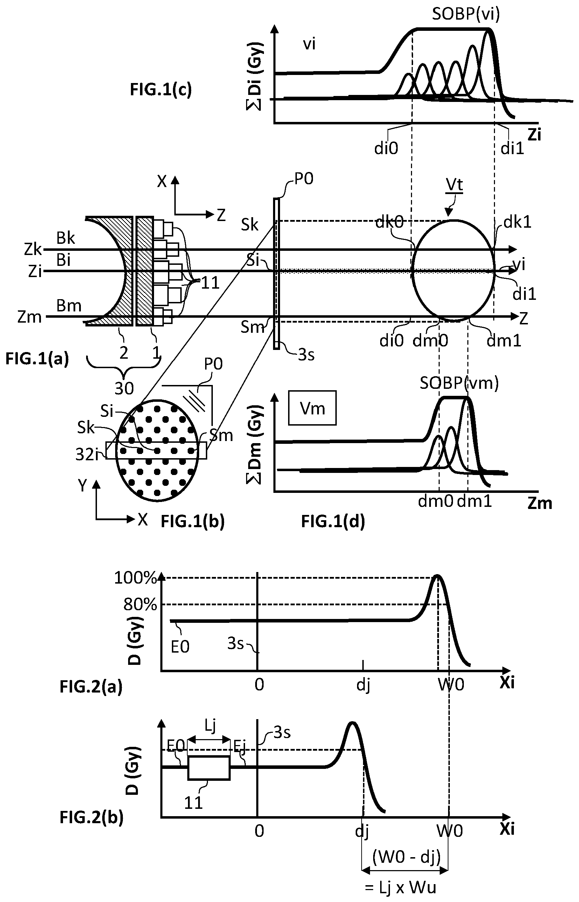

The present invention concerns a dynamic shaping device (30) that can be re-used and re-configured to shape the SOBP's of pencil beams matching the dose deposition patterns defined by treatment plans for the treatment of target volumes (Vt) of different geometries. A target area (At) is defined by projecting the target volume (Vt) onto a plane P0 defined by an X-axis and a Y-axis perpendicular to a central irradiation axis (Z). An array of spots (Sj) is defined, distributed on the plane (P0) covering at least the whole of the target area (At) as illustrated in Figures 1(b), 3(a), and 12(a) to 12(c). The dynamic shaping device (30) of the present invention comprises a dynamic ridge filter (1) and / or a dynamic range shifter (2).

### RIDGE FILTER (1)

The dynamic ridge filter (1) of the present invention is configured for shaping a dose deposition zone by radiation with charged particles pencil beams, preferably with proton pencil beams, and for depositing doses (Dj) applied by pencil beams (Bj) propagating along corresponding irradiation axes (Zj) fanning out of a central irradiation axis (Z) with a pencil beam scanning (PBS) system. The pencil beams are delivered into a sequence of specific volumes (Vj) along an X-axis to form specific volume stripes, which are distributed next to one another along a Y-axis transverse, preferably normal, to the X-axis. The specific volumes (Vj) define in combination the target volume (Vt) comprising the tumoral cells.

The dynamic ridge filter (1) comprises a number of energy spreading units (11), each energy spreading unit (11) being characterized by a corresponding energy spreading capacity for spreading the dose deposited along the irradiation axis (Zj) by the corresponding pencil beams (Bj) traversing one or more energy spreading units (11).

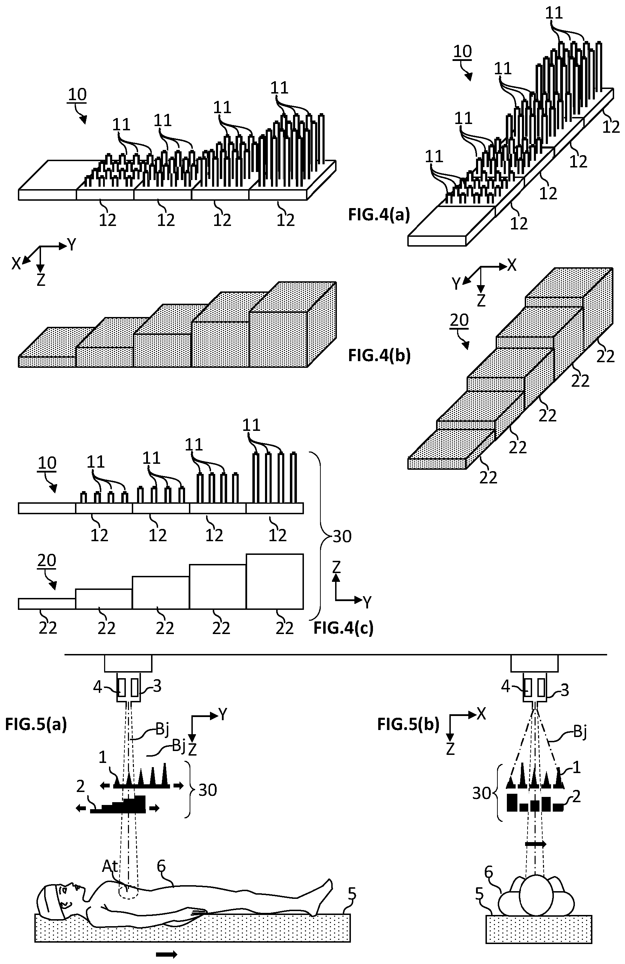

The gist of the present invention is that the ridge filter is dynamic, in that a topography of the ridge filter can be varied according to the requirements of the treatment plan. This result is achieved as follows. The energy spreading units (11) are distributed in filter modules (12). Each filter module (12) supports one or more energy spreading units (11) and has an area normal to the central irradiation axis (Z) compatible with a cross-sectional diameter (D100) of the corresponding pencil beam (Bj). As shown in Figures 4(a) and 4(c), the filter modules (12) are arranged in different filter rows (10), each extending along the row-direction (Y). Each filter row (10) comprises a number of dissimilar filter modules (12), each filter module of a filter row (10) having a different energy spreading capacity than the other filter modules (12) of the same filter row (10).

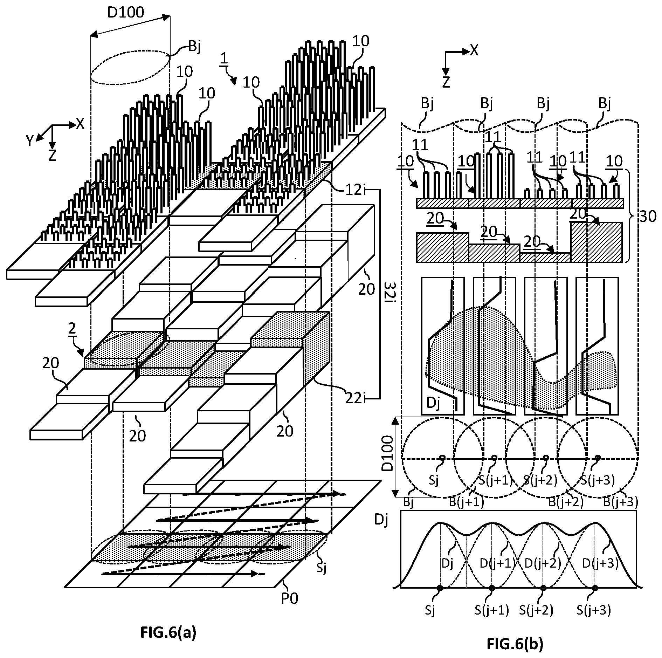

The ridge filter of the present invention is dynamic because each filter row can be displaced independently to form a filter string (12i) along the X-axis as shown in Figure 6(a). With this construction, the filter string is configured to shape the dose deposition in the specific volumes (Vj) forming the specific volume stripe facing the filter string along the irradiation axes (Zj).

All filter rows (10) are preferably identical to one another and comprise a same selection of filter modules (12). Each module is to the filter string (12i) what letters are to a word. They can be combined in different arrangements to define different "words". As shown in Figures 6(a) and 6(b), each module (12) preferably has dimensions of the same order of magnitude as the diameter (D100) of the pencil beams.

### ENERGY SPREADING UNITS (11)

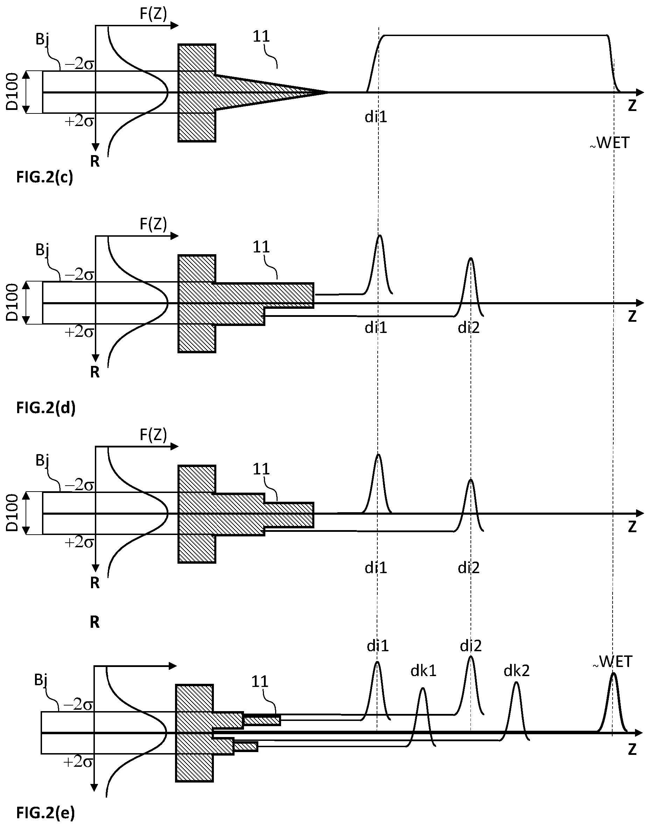

Each filter module (12) comprises one or more energy spreading units (11) supported on a base of the filter modules (12). As shown in Figures 2(a) and 2(b), by interposing an energy spreading unit (11) on the path of a hadron beam, the energy thereof can be controlled for shortening the propagation distance thereof through a body (e.g., water or tissues of a patient). It is thus possible to displace the position of the Bragg Peak of hadron particles where the full dose (Dj) of each particle is deposited, without varying the energy of the hadron beam emitted out of the nozzle (3). Figures 2(c) to 2(e) show how the positions of the Bragg peaks of one or more pencil beam can be controlled by passing them through different embodiments of energy spreading units (11). A pencil beam (Bj) has a diameter (D100), which can be defined, e.g., as a function of the variance of the Gaussian distribution of particles of the pencil beam (Bj) (for example, in Figures 2(c) to 2(e), D100 = 4 *σ*, wherein σ² is the variance of the Gaussian distribution). If the pencil beam traverses different thicknesses of the material forming the energy spreading units (11), as many different Bragg peaks are obtained which, put together form an SOBP. By calculating precisely the contribution of each shifted pencil beam, a smooth SOBP can be obtained.

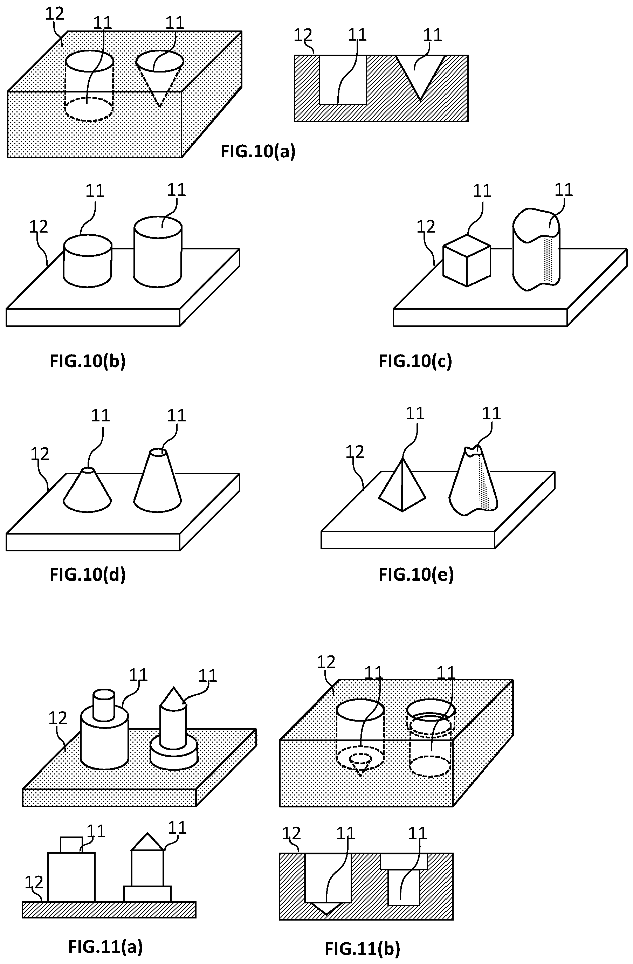

In an embodiment shown in Figures 10(b) and 10(c), the energy spreading units (11) can be in the form of spikes of generalized cylindrical, preferably prismatic geometry. A generalized cylindrical geometry is defined by parallel lines (= a generatrix) passing by the perimeter of a planar base of any geometry, preferably albeit not necessarily circular. A prismatic geometry is herein defined as a cylindrical geometry whose planar base is polygonal. Alternatively, as shown in Figures 10(d) and 10(e), the energy spreading units (11) can have a conical geometry, truncated or not, preferably a pyramidal geometry. A conical geometry is defined by straight lines passing by an apex and by the perimeter of a planar base of any geometry, preferably albeit not necessarily circular. A pyramidal geometry is a conical geometry whose planar base is polygonal.

As shown in Figures 5(a) and 5(b), the pencil beams (Bj) exit out of a nozzle (3) which can be considered as forming the apex of a scanning triangle in a (X, Z) plane and of a scanning cone whose base encloses at least the target area (At) on the patient 6, depending on whether the electromagnetic elements (4) are configured for deviating the pencil beams (Bj) along the X-axis only or along both X- and Y-axes. The central irradiation axis (Z) is defined by the axial axis of the nozzle. In the case of scanning along both X-and Y-axes, the pencil beams can scan within the scanning cone and thus cover the whole target area (At). If the tumour is too large to allow the beamlets to scan over the whole corresponding target area (At), the irradiation session must be carried out in two or more stages, including moving the nozzle to cover the whole target area. Since the distance of the nozzle to the plane (P0) is substantially larger than a diameter of the target area (At), the pencil beams (Bj) can be approximated to be about parallel to the central irradiation axis (Z), albeit this is not strictly true, because of the aperture of the scanning cone. This aperture is, however, very small and the irradiation axes (Zj) can be considered as approximately parallel to the central irradiation axis (Z). This issue is well known to the skilled person. For sake of clarity and conciseness, in continuation, it is approximated that the irradiation axes are parallel to the central irradiations axis (Z).

In an alternative embodiment illustrated in Figure 10(a), the energy spreading units (11) can be in the form of orifices of generalized cylindrical or conical geometry penetrating in a support base of the filter modules (12). Each orifice extends from an aperture opening at a surface of the support base and penetrating to a given depth leaving a resulting thickness of material of the support base. As for the spikes discussed supra, it is preferred that the orifices be configured in use for having a major axis of the cavities extending parallel to the corresponding irradiation axes (Zj).

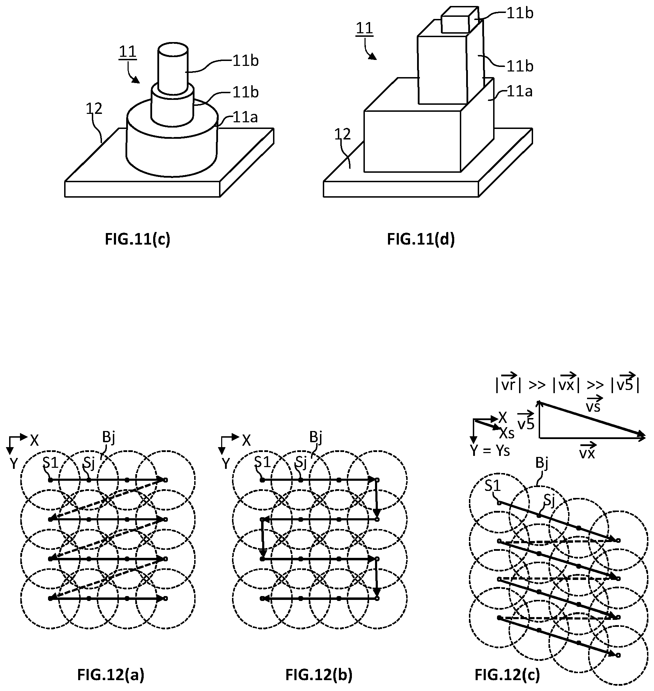

As illustrated in Figures 11(a) to 11(d), each filter module (12) can comprise one or more energy spreading units (11), each formed by a plurality of spreading subunits (11a-11c) having differing cross-sectional areas (Ai) normal to the irradiation axis (Zj). The spreading subunits (11a-11c) are stacked on top of one another along a major axis of the energy spreading unit (11) configured in use for extending parallel to the irradiation axis (Zj). By using energy spreading units formed by stacking spreading subunits (11a-11c) having different energy spreading capacities, a corresponding SOBP can be defined as described in EP2021/208699.

### DYNAMIC RANGE SHIFTER (2)

The dynamic shaping device (30) of the present invention also includes a range shifter (2) configured for shaping a dose deposition zone by radiation with charged particles beams, preferably with proton beams, and for depositing doses (Dj) applied by pencil beams (Bj) propagating along corresponding irradiation axes (Zj) fanning out of a central irradiation axis (Z) with a pencil beam scanning (PBS) system and delivered into a sequence of specific volumes (Vj) along a X-axis to form specific volume stripes distributed next to one another along a Y-axis transverse, preferably normal, to the X-axis. The specific volumes (Vj) define in combination the target volume (Vt) comprising the tumoral cells.

The range shifter (2) has a thickness of material measured along the central irradiation axis (Z) which varies over an area of the range shifter (2). In use, the area is preferably planar and preferably configured for being substantially normal to the corresponding irradiation axes (Zj). The thickness of range shifting material determines the corresponding amount of absorbed energy (ΔE) of the pencil beam (Bj) of the charged particles traversing the thickness

The gist of the present invention is that the range shifter (2) is dynamic, in that a topographic thickness of the range shifter can be varied according to the requirements of the treatment plan. This result is achieved as follows. The range shifter comprises shifter modules (22) arranged side by side to form shifter rows (20), as illustrated in Figure 4(b). Each shifter row (20) comprises a selection of shifter modules (22) having a constant thickness different from the other shifter modules (22) of the same shifter row (20) and configured for absorbing different amounts of energy (ΔE) from the pencil beam (Bj).

As shown in Figure 6(a), all shifter rows (20) can be displaced independently to form a shifter string (22i) along the X-axis. As shown in Figure 6(b), the shifter string is configured to shape along the irradiation axes (Zj) the dose deposition in the specific volumes (Vj) forming the specific volume stripe facing the shifter string along the irradiation axes (Zj). In particular, the range shifter defines the geometry of the furthest downstream end of the dose deposition profile.

All shifter rows (20) are preferably identical and comprise a same selection of shifter modules (22). Each shifter module is formed by a plate of preferably constant thickness and of dimensions on the plane (X, Y) of the same order of magnitude as the pencil beam diameter (D100) and as the filter modules (12).

### DYNAMIC SHAPING DEVICE (30) AND TREATMENT STATION

As shown in Figures 5(a)&5(b) and 6(a)&6(b), the combination of at least a dynamic ridge filter (1) as defined supra and at least a dynamic range shifter (2) as defined supra. disposed in a sequence along a beam path of a pencil beam (Bj) forms a dynamic shaping device (30). The dynamic ridge filter (1) and range filter (2) are superimposed such that the filter string (12i) and the shifter string (22i) face each other along a direction, which in use is parallel the irradiation axis (Zj), to form in combination a radiation string (32i). This is illustrated in Figure 6(a) with the shaded filter string (12i) and shifter string (22i).

The ridge filter (1) can indifferently be positioned upstream or downstream of the range shifter (2) relative to the particles flow direction. The filter rows (10) and shifter rows (20) must be translated along the Y-axis such as to bring the right sequence of filter modules (12) and shifter modules (22) aligned along the X-axis to define the required radiation string (32i) matching the dose deposition pattern onto the corresponding specific volume stripe as defined in the treatment plan. The filter rows (10) and shifter rows (20) must be translated again to define the required radiation string (32i) matching the dose deposition pattern onto a next corresponding specific volume stripe adjacent to the first one along the Y-axis, as defined in the treatment plan.

The present invention also concerns a treatment station for shaping a dose deposition zone by radiation with charged particles beams, preferably with proton beams, and for depositing doses (Dj) applied by pencil beams (Bj) propagating along corresponding irradiation axes (Zj) fanning out of a central irradiation axis (Z) with a pencil beam scanning (PBS) system and delivered into a sequence of specific volumes (Vj) along a X-axis to form specific volume stripes distributed next to one another along a Y-axis transverse, preferably normal, to the X-axis, the specific volumes (Vj) defining in combination a target volume (Vt) comprising the tumoral cells. As illustrated in Figures 5(a) and 5(b), the treatment station comprises,
- a source of pencil beams (Bj) of accelerated charged particles, preferably of protons, and
- a nozzle (3) for directing the pencil beams (Bj) of accelerated charged particles towards the target volume (Vt), the nozzle comprising electromagnetic elements (4) configured for deviating the pencil beam (Bj) to scan along at least the X-axis as the nozzle remains static, and
- a couch (5) for receiving the patient (6), and
- a dynamic ridge filter according to the present invention, and / or
- a range shifter (2) according to the present invention, and
- one or more processors configured for controlling various components of the treatment station,

Preferably, the treatment station comprises both dynamic ridge filter (1) and dynamic range shifter (2) according to the present invention and are aligned such that the filter string (12i) and the shifter string (22i) face each other along a direction, which in use is parallel the irradiation axis (Zj), to form in combination a radiation string (32i).

### USE OF THE TREATMENT STATION

The PBS system is configured for pointing a pencil beam (Bj) over each spot (Sj) in a given sequence. The spot sequence is preferably composed of a series of scans along the X-axis repeated over i = 1 to m radiation strings (32i) distributed along the Y-axis. As moving the nozzle is most time consuming, it is preferred to carry out a whole portion of a radiation sequence with the nozzle (3) remaining static. As shown in Figures 5(a)&5(b) and 13(a)&13(b) to 15(a)&15(b), the nozzle (3) is provided with electromagnetic elements (4) configured for deviating the pencil beams (Bj) at least along the X-axis, preferably along both X-axis and Y-axis.

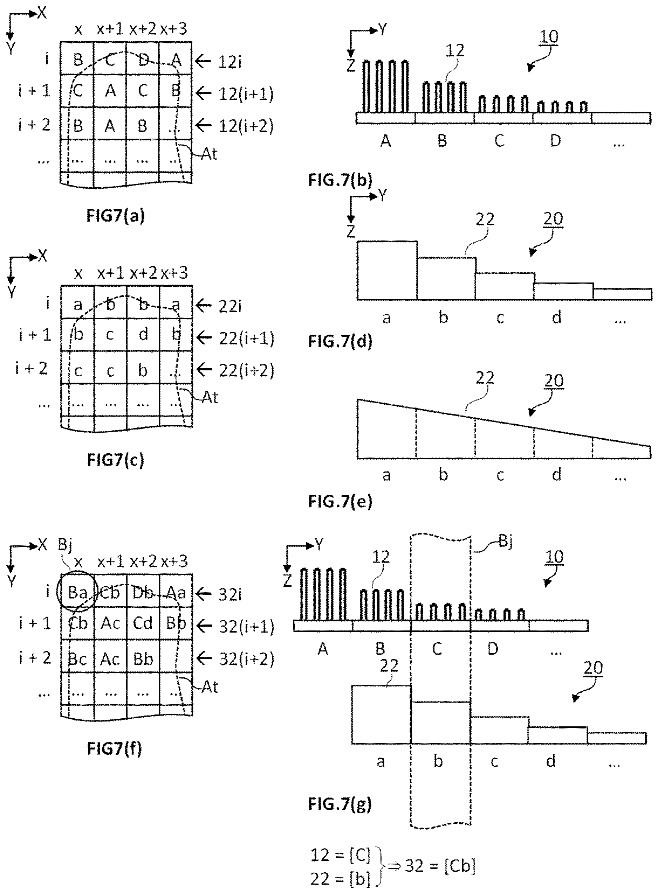

To carry out a radiation treatment session with a radiation station according to the present invention, the one or more processors can be configured for controlling the actions illustrated in Figures 7(a), 7(c), 7(f), 8(a) to 8(d) and 9(a) to 9(d) discussed in continuation.

Figure 7(a) shows an example of a spot pattern covering a target area (At), with a series of filter strings (12i) for different positions (i = 1 to ...) along the Y-axis. The letters A, B, C, D refer to a specific filter module (12) as defined in Figure 7(b). Figure 7(c) shows the same spot pattern as in Figure 7(a), with series of shifter strings (22i) for different positions (i = 1 to ...) along the Y-axis, wherein the letters a, b, c,... refer to specific shifter modules (22) as defined in Figures 7(d) and 7(e). As shown in Figures 4(b), 4(c), 6(a), and 7(d), the shifter row (20) is preferably formed of discrete shifter modules (22) each of substantially constant thickness. In alternative embodiments, the shifter row (20) can have a continuous slope as illustrated in Figure 7(e). The size of a shifter module is defined by the diameter of the pencil beam (Bj) as can be deduced from Figure 7(g). A radiation string (32i) is formed by the combination of a filter string (12i) and a shifter string (22i) as shown in Figure 7(f). A radiation module (32) is obtained by sliding along the Y-axis each filter row (10) and shifter row (20) of the dynamic shaping device (30) to align the required filter modules (12) and shifter modules (22) with the corresponding pencil beams (Bj). In Figures 7(a) and 7(c), the modules of a filter row (10) are identified by letters A, B, C, D and the ones of a shifter row are identified by letters, a, b, c, d. The radiation modules (32) can be formed by any combinations of the filter modules (12) and shifter modules (22), with a total of 16 combinations Aa, Ab, Ac, Ad, Ba, Bb, Bc... For example, Figure 7(g) shows the alignment of the filter row (10) and shifter row (20) to align filter module (12 = B) with shifter module (22 = a) to yield the radiation module (32 = [Ba]) required at spot (Sj) located at (i, j) in Figure 7(f).

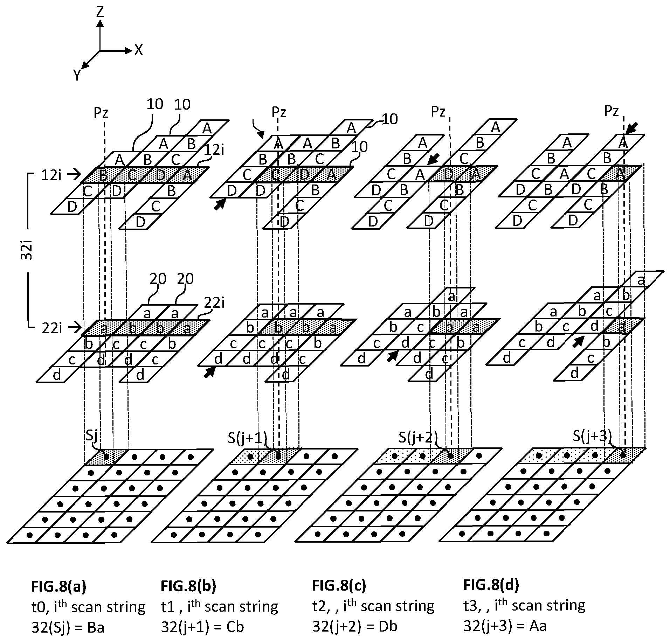

As shown in Figure 8(a), the filter rows (10) and the shifter rows (20) are moved independently along the Y-axis to yield a sequence of filter modules (12) and shifter modules (22) forming in combination an i^{th} radiation string (32i) composed of an i^{th} filter string (12i) and of an i^{th} shifter string (22i) along the X-axis according to a predefined treatment plan illustrated in Figures 7(a), 7(c), and 7(f). The beam paths of the pencil beams (Bj) of an i^{th} scanning string sequentially traverse the corresponding radiation modules (32) of the i^{th} radiation string (32i) before attaining the target volume (Vt). This corresponds to any one sequence defined in line i, i+1, i+2... in Figure 7(f).

The electromagnetic elements (4) are controlled for orienting the pencil beam (Bj) through a first filter module (12) of the i^{th} filter string (12i) and through a first shifter module (22) of the i^{th} shifter string (22i), and towards a first spot (Sj) of the i^{th} scanning string formed by a sequence of spots (Sj) distributed along the X-axis. After the pencil beam (Bj) traversing the first spot (Sj) of the i^{th} scanning string delivered a predefined dose (Dj), the electromagnetic elements (4) are controlled for sequentially orienting the pencil beams (Bj) of the i^{th} scanning string through each one of the sequence of filter modules (12) forming the i^{th} filter string and through each one of the sequence of shifter modules (22) forming the i^{th} shifter string, and towards the sequence of spots (Sj) of the i^{th} scanning string. As shown in Figure 8(a), the radiation sequence starts by pointing a j^{th} pencil beam (Bj) to a j^{th} spot (Sj) on the i^{th} scanning string (i) of spots distributed along the X-axis. The radiation string (32i) formed by a combination of the filter string (12i) and the shifter string (22i) is defined by the i^{th} scanning string (32i) = [Ba]-[Cb]-[Db]-[Aa] illustrated in Figure 7(f). The j^{th} pencil beam (Bj) passes through the radiation module (12) = [Ba] to deposit a first predefined fraction of the dose (Dj) to be deposited onto the specific volume (vi) associated with the j^{th} spot (Sj). As shown in Figures 8(b) to 8(d), the electromagnetic elements (4) are activated to deviate the pencil beam towards a (j+1)^{th} spot (S(j+1)) adjacent to the j^{th} spot (Sj) along the X-axis. As the (j+1)^{th} pencil beam passes trough the radiation module (32) = [Cb] a first predefined fraction of the dose (Dj) is deposited onto the specific volume (vi) associated with the (j+1)^{th} spot (Sj). During this time, the previous filter row (12) and shifter row (22) facing the first spot (Sj) are translated to bring the radiation module (32) = [Cb] as defined in Figure 7(f) for the first spot of the (i +1)^{th} scanning string adjacent to the i^{th} scanning string along the Y-axis.

These operations are repeated for each spot of the i^{th} filter string (12i) until the last spot (S(j+3)) thereof is irradiated by a (j+3)^{th} pencil beam (B(j+3)), to deposit a first predefined fraction of the dose (Dj) to be deposited onto the specific volume (vi) associated with the (j+3)^{th} spot (S(j+3)). Each time, after a spot of the i^{th} scanning string was irradiated, the filter row (10) and shifter row (20) facing the last irradiated spot are translated to bring the radiation module (32) as defined in Figure 7(f).

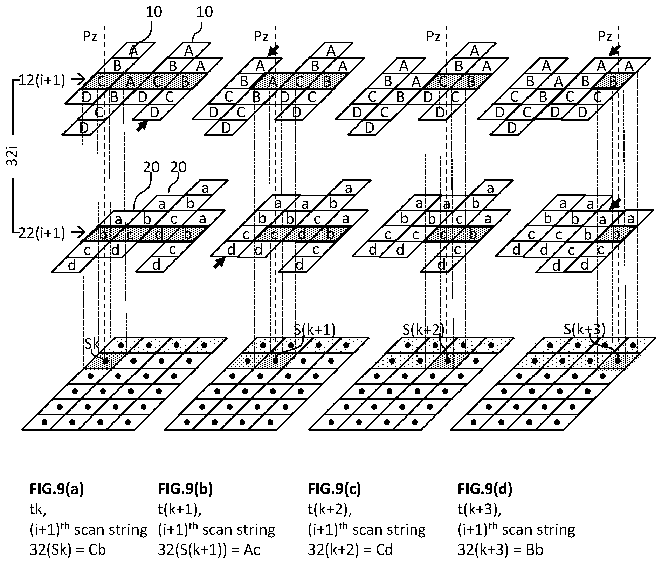

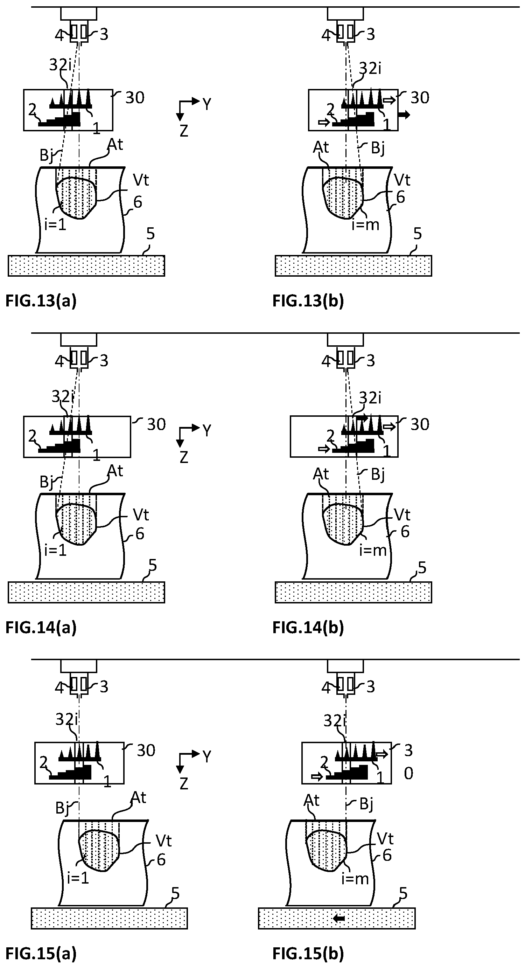

The next pencil beam is oriented towards a first spot of a next scanning string (i+1) of spots. This can be achieved in different manners described below with reference to Figures 13(a)&13(b) to 15(a)&15(b). The previous steps are repeated for all the spots aligned along the (i+1)^{th} string as shown in Figures 9(a) to 9(d). And so on, until all spots aligned along all scanning strings (i, i+1...) extending along the X-axis and distributed along the Y-axis have been irradiated. Moving the filter rows (10) (and shifter rows (20)) to their respective positions required by the corresponding spot of the next scanning string as soon as the corresponding spot has been irradiated is advantageous in that the shaping device (30) is sequentially modifying the radiation string (32i) for the radiation string required for shaping the pencil beams pointed at the spots of the next scanning string. This way, the flow of accelerated particles needs not be interrupted until the new radiation string has been formed before starting scanning along an (i+1)^{th} scanning string. This is illustrated by comparing Figures 8(a) to 8(d) showing the i^{th} radiation string [Ba]-[Cb]-[Db]-[Aa] with Figures 9(a) to 9(d) showing the (i+1)^{th} scanning string [Cb]-[Ac]-[Cd]-[Bb] as defined in Figure 7(f), ready for irradiating the target volume along the (i+1)^{th} scanning string. When the j^{th} spot (Sj) of the i^{th} scanning string has been irradiated through the radiation string (32) = [Aa], the one or more processing units control the electromagnetic elements (4) to move the beam (Bj) to the (j+1)^{th} spot (S(j+1)) and control the dynamic shaping device (30) to move the x^{th} filter row (10) and the x^{th} shifting row (20) along the Y-axis, such as to align the filter and shifting modules (12, 22) to yield the radiation module (32) = [Cb] required for the j^{th} spot of the (i+1)^{th} scanning string as defined in Figure 7(f). The same process is applied after a dose has been deposited in the specific volumes associated with each successive spot (Sj, S(j+1),...) of the i^{th} scanning string.

### PASSING FROM AN i^{th} TO AN (i+1)^{th} SCANNING STRING

Figures 12(a) to 12(c) show three possible embodiments of spots irradiation sequences, all including a scan comprising at least a component along the X-axis. Figure 12(a) illustrates a reading sequence wherein as a scan along the X-axis is completed, the pencil beam is returned to the initial position one string below, as when reading a document. Alternatively, Figure 12(b) illustrates a scarf-sequence wherein as a scan along the X-axis is completed, the pencil beam jumps to the closest spot located on the next string. The choice between the reading sequence or the scarf sequence depends on many parameters which extend beyond the scope of the present invention, including the geometry of the target volume, the position of the spots to be irradiated at ultra-high dose deposition rates, the performance of the source of accelerated particles, the performance of the scanning system, and the like.

Figure 12(c) shows a reading sequence obtained when the couch (5) supporting the patient (6) translates along the Y-axis. The combination of the scanning of the pencil beam along the X-axis at an X-rate (vx) and the translation of the couch (5) and patient (6) along the Y-axis at a couch rate (v5, with vx >> v5) yields a scanning direction (xs) at a resulting scanning rate (vs = (vx² + v5²)^{1/2}). The angle formed by the scanning direction (xs) with the X-axis illustrated in Figure 12(c) is therefore largely exaggerated. Note that the return rate (vr) from the last spot of a given scanning string to the first spot of a next scanning string is much higher than the X-rate (vx).

Scanning from spot to spot along a scanning string parallel to the X-axis is controlled by the electromagnetic elements (4) at the level of the nozzle (3). Passing from an i^{th} scanning string to an (i+1)^{th} scanning string can be carried out in different ways illustrated in Figures 13(a)&13(b) to 15(a)&15(b)..

In a first embodiment illustrated in Figures 13(a)&13(b) and 14(a)&14(b), to allow the whole of the target area (At) to be scanned by the pencil beams (Bj), the electromagnetic elements (4) are configured for deviating the pencil beam (Bj) to scan also along the Y-axis as the nozzle remains static. State of the art ridge filters and range shifters (i.e., non-dynamic) are designed to intercept the pencil beams (Bj) as they scan along both X- and Y-axes. This is not the case with the dynamic ridge filter (1), range shifter (2), and resulting shaping device (30) of the present invention, since only the modules aligned along the radiation string (32i) are arranged to yield SOBP's according to the treatment plan. As the pencil beams shift along the Y-axis they do not face the radiation string (32i) anymore. Two solutions are proposed to ensure that the pencil beams traverse the dynamic shaping device through the radiation string with the right arrangement of modules (12, 22) to yield the defined SOBP's over the whole target volume. Either the whole shaping device (30) moves along the Y-axis together with the radiation string (32i) and the pencil beams (Bj) or the radiation string (32i) moves relative to the shaping device (32) along the Y-axis with pencil beams (Bj).

Figures 13(a)&13(b) illustrate two scanning sequences at a first scanning string (i = 1 in Figure 13(a)) and a last scanning string (i = m in Figure 13(b)). The electromagnetic elements (4) are configured to deviate the pencil beam (Bj) from the first (i = 1) to the last (i = m) scanning strings. To ensure that the pencil beams traverse the radiation string (32i) for all scanning strings distributed along the Y-axis, the whole shaping device (30) can move along the Y-axis together with the pencil beams (Bj). The filter rows (10) and shifter rows (20) can be moved independently to ensure that the right arrangement of modules (12, 22) is present as a pencil beam (Bj) traverses the radiation string (32i) which is always aligned with the pencil beams (32i). This solution is quite straightforward and requires little processing power to coordinate the translations along the Y-axis of the shaping device (30) and of the pencil beams (Bj).

In an alternative solution illustrated in Figures 14(a) & 14(b) which, like Figures 13(a) and 13(b), show two scanning sequences at a first scanning string (i = 1 in Figure 14(a)) and a last scanning string (i = m in Figure 14(b)). The electromagnetic elements (4) are configured to deviate the pencil beam (Bj) from the first (i = 1) to the last (i = m) scanning strings. To ensure that the pencil beams traverse the radiation string (32i) for all scanning strings distributed along the Y-axis, the radiation string (32i) (illustrated with a rectangular window in Figures 13 to 15) moves relative to the shaping device along the Y-axis together with the deviation of the pencil beams (Bj) along a new scanning string. This is quite simple to achieve and requires little processing power to coordinate the movements of the filter / shifter rows (10, 20) to align the right arrangements of modules along radiations strings that move together with the pencil beams along the Y-axis.

In an alternative embodiment illustrated in Figures 15(a) and 15(b), the electromagnetic elements (4) do not deviate the pencil beams (Bj) along the Y-axis. The shaping device (30) does not move. The couch (5) supporting the patient (6), however, is provided with a translation system configured for translating the couch along the Y-axis to align the specific volumes (Vj) with the corresponding pencil beams (Bj). The movement of the couch (5) can be continuous at a couch rate (v5) or it can be sequential, moving only when the pencil beams are deviated along the Y-axis. It is preferred that the couch moves continuously at a couch rate (v5) yielding a scanning sequence as for example, illustrated in Figure 13(c).

A combination of the solutions illustrated in Figures 13(a) & 13(b) to 15(a) & 15(b) can also be envisaged to ensure that the pencil beam traverse the right sequence of modules (12, 22) at each spot (Sj) covering the whole target area (At).

### ADVANTAGES OF THE PRESENT INVENTION

The dynamic shaping device (30) of the present invention comprises a dynamic ridge filter and a dynamic range shifter comprising a number of filter rows (10) and a number of shifter rows, respectively. Each filter row is composed of a same selection of filter modules (12) of different energy spreading properties distributed along a length of the filter row parallel to the Y-axis. Similarly, each shifter row (20) is composed of a same selection of shifter modules (22) of different range shifting properties distributed along a length of the shifter row parallel to the Y-axis.

The filter rows and shifter rows can be translated along the Y-axis independently of one another to yield radiation modules (32) formed by a filter module and a shifter module aligned along a corresponding irradiation axis (Z). By translating the filter rows and shifter rows during scanning of the pencil beams, the dynamic shaping device (30) adapts dynamically to a predefined treatment plan.

The present invention is very advantageous over the prior art shaping devices because a single dynamic shaping device (30) according to the present invention can be used several times to treat different patients, or a same patient at different moments in time corresponding to different radiation sessions. The dynamic shaping device of the present invention can be adapted to match the treatment plans associated with the treatment of target volumes of different geometries. It can also be adapted for us with particles accelerators of different performance. Not having to manufacture a new ridge filter and a new range shifter before every radiation session is a substantial time saving factor.

| **REF #** | **Feature** |
|---|---|
| 1 | Dynamic ridge filter |
| 2 | Dynamic range shifter |
| 3 | Nozzle |
| 4 | Electromagnetic element |
| 5 | Couch |
| 6 | Patient |
| 10 | Filter row |
| 11 | Energy degrading element |
| 11a-11c | Spreading subunit |
| 12 | Filter module |
| 12i, i=1-k | Filter string |
| 20 | Shifter row |
| 22 | Shifter module |
| 22i, i=1-k | Shifter string |
| 30 | Shaping device |
| 32 | Radiation module |
| 32i, i=1-k | Radiation string |
| Pz | Beam |
| Si.j | Spot |
| SOBP | Spread Out Bragg Peak |
| Vt | Target volume |
| X | X-axis defining the scanning direction |
| Xs | Stripe direction |
| Y | Y-axis defining the row direction |
| Ys | Column direction |
| Z | Direction normal to plane (X, Y) |
| Zj | Irradiation axis |

## Claims

1. Dynamic ridge filter (1) for shaping a dose deposition zone by radiation with charged particles beams, preferably with proton or other light ion beams, and for depositing doses (Dj) applied by pencil beams (Bj) propagating along corresponding irradiation axes (Zj) fanning out of a central irradiation axis (Z) by a pencil beam scanning (PBS) system and delivered in sequence to specific volumes (Vj) along a X-axis to form specific volume stripes distributed next to one another along a Y-axis transverse, preferably normal, to the X-axis, the specific volumes (Vj) defining in combination a target volume (Vt) comprising the tumoral cells,
wherein the dynamic ridge filter comprises a number of energy spreading units (11), each energy spreading unit (11) being **characterized by** a corresponding energy spreading capacity for spreading the dose deposited along the irradiation axis (Zj) by the corresponding pencil beams (Bj) traversing one or more energy spreading units (11),
**Characterized in that,**
• the energy spreading units (11) are distributed in filter modules (12), each filter module (12) supporting one or more energy spreading units (11) and having an area normal to the central irradiation axis (Z) compatible with a cross-section of the corresponding pencil beam (Bj), wherein the filter modules (12) are arranged in different filter rows (10), each extending along the row-direction (Y),
• each filter row (10) comprises a number of dissimilar filter modules (12), each filter module of a filter row (10) having a different energy spreading capacity from the other filter modules (12) of the same filter row (10), wherein
∘ each filter row can be displaced independently to form a filter string (12i) along the X-axis and
∘ the filter string is configured to shape the dose deposition in the specific volumes (Vj) forming the specific volume stripe facing the filter string along the irradiation axes (Zj).

2. Dynamic ridge filter (1) according to claim 1, wherein the energy spreading units (11) are in the form of spikes of generalized cylindrical, preferably prismatic geometry, or of conical geometry, truncated or not, preferably a pyramidal geometry supported on a base of the filter modules (12) and configured in use for having a major axis of the spikes extending parallel to the corresponding pencil beam axes (Zj).

3. Dynamic ridge filter (1) according to claim 1, wherein the energy spreading units (11) are in the form of orifices of generalized cylindrical or conical geometry penetrating in a support base of the filter modules (12), each orifice extending from an aperture opening at a surface of the support base and penetrating to a given depth leaving a resulting thickness of material of the support base, and configured in use for having a major axis of the cavities extending parallel to the corresponding pencil beam axes (Zj).

4. Dynamic ridge filter (1) according to any one of the preceding claims, wherein each filter module (12) comprises one or more energy spreading units (11) each formed by a plurality of spreading subunits (11a-11c) having differing cross-sectional areas (Ai) normal to the irradiation axis (Zj), and stacked on top of one another along a major axis of the energy spreading unit (11) configured in use for extending parallel to the irradiation axis (Zj), wherein all spreading subunits (11a-11c) of the stack have different energy spreading capacities.

5. Dynamic ridge filter (1) according to any one of the preceding claims, wherein all filter rows (10) are identical and comprise a same selection of filter modules (12).

6. Dynamic range shifter (2) for shaping a dose deposition zone by radiation with charged particles beams, preferably with proton or other light ion beams, and for depositing doses (Dj) applied by pencil beams (Bj) propagating along corresponding irradiation axes (Zj) fanning out of a central irradiation axis (Z) by a pencil beam scanning (PBS) system and delivered in sequence to specific volumes (Vj) along a X-axis to form specific volume stripes distributed next to one another along a Y-axis transverse, preferably normal, to the X-axis, the specific volumes (Vj) defining in combination a target volume (Vt) comprising the tumoral cells,
wherein the range shifter (2) has a thickness of material varying over an area of the range shifter (2), the area being configured for being substantially normal to the irradiation axis (Zj) in use, wherein a value of the thickness determines a corresponding amount of absorbed energy (ΔE) of the pencil beam (Bj) of the charged particles traversing the thickness,
**Characterized in that,**
• The range shifter comprises shifter modules (22) arranged side-by-side to form shifter rows (20), wherein each shifter row (20) comprises a selection of shifter modules (22) having a constant or almost constant thickness different from the other shifter modules (22) of the same shifter row (20), and configured for absorbing different amounts of reference absorbed energy (ΔE) from the pencil beam (Bj), wherein
• each shifter row (20) can be displaced independently to form a shifter string (22i) along the X-axis,
• the shifter string is configured to shape along the irradiation axes (Zj) the dose deposition in the specific volumes (Vj) forming the specific volume stripe facing the shifter string along the irradiation axes (Zj).

7. Dynamic range shifter (2) according to claim 6, wherein all shifter rows (20) are identical and comprise a same selection of shifter modules (22).

8. Dynamic shaping device (30) for shaping a dose deposition zone by radiation with charged particles beams, preferably with proton beams, and for depositing doses (Dj) applied by pencil beams (Bj) propagating along corresponding irradiation axes (Zj) fanning out of a central irradiation axis (Z) with a pencil beam scanning (PBS) system and delivered into a sequence of specific volumes (Vj) along a X-axis to form specific volume stripes distributed next to one another along a Y-axis transverse, preferably normal, to the X-axis, the specific volumes (Vj) defining in combination a target volume (Vt) comprising the tumoral cells,
**Characterized in that,** it comprises at least a dynamic ridge filter (1) according to any one of claim 1 to 5 and at least a dynamic range shifter (2) according to claim 6 or 7. disposed in a sequence along a beam path of a pencil beam (Bj), such that the filter string (12i) and the shifter string (22i) face each other along a direction, which in use is parallel the irradiation axis (Zj), to form in combination a radiation string (32i).

9. Treatment station for shaping a dose deposition zone by radiation with charged particles beams, preferably with proton beams, and for depositing doses (Dj) applied by pencil beams (Bj) propagating along corresponding irradiation axes (Zj) fanning out of a central irradiation axis (Z) with a pencil beam scanning (PBS) system and delivered into a sequence of specific volumes (Vj) along a X-axis to form specific volume stripes distributed next to one another along a Y-axis transverse, preferably normal, to the X-axis, the specific volumes (Vj) defining in combination a target volume (Vt) comprising the tumoral cells, wherein the treatment station comprises,
• a source of pencil beams (Bj) of accelerated charged particles, preferably of protons,
• a nozzle (3) for directing the pencil beams (Bj) of accelerated charged particles towards the target volume (Vt), the nozzle comprising electromagnetic elements (4) configured for deviating the pencil beam (Bj) to scan along at least the X-axis as the nozzle remains static,
• a ridge filter,
• a range shifter,
• a couch or chair (5) for receiving the patient in supine, prone, seated or standing position,
• one or more processors configured for controlling various components of the treatment station,
**Characterized in that,**
• the ridge filter is a dynamic ridge filter (1) according to any one of claims 1 to 5,
• the range shifter is a dynamic range shifter (2) according to claim 6 or 7,
wherein the dynamic ridge filter (1) and dynamic range shifter (2) are arranged such as to form a shaping device according to claim 8.

10. Treatment station according to claim 9, wherein either,
• the electromagnetic elements (4) are configured for deviating the pencil beam (Bj) to scan also along the Y-axis as the nozzle remains static, and
∘ the shaping device (30) is provided with a translation system configured for following a translation of the pencil beam along the Y-axis such that the filter string (12i) and the shifter string (22i) move together with the shaping device (30) keeping in alignment with the pencil beam along the Y-axis, or
∘ the filter string (12i) and the shifter string (22i) move along the Y-axis relative to the shaping device (30) which preferably remains static relative to the target volume (Vt), in order to keep in alignment with the pencil beam along the Y-axis, or
• The couch (5) is provided with a translation system configured for translating the couch along the Y-axis to align the specific volumes (Vj) with the corresponding pencil beams (Bj), whilst the shaping device (30) and the filter string and shifter string preferably remain static relative to the target volume, or.
• a combination of the foregoing options.

11. Treatment station according to claim 9 or 10, wherein the one or more processors are configured for:
• moving the filter rows (10) and the shifter rows (20) along the Y-axis to yield a sequence of filter modules (12) forming an i^{th} filter string (12i) and of an i^{th} shifter string (22i) extending along the X-axis to yield an i^{th} radiation string (32i) according to a predefined treatment plan, such that the beam paths of the pencil beams (Bj) of an i^{th} scanning string traverse corresponding radiation modules (32) of the sequence of radiation modules (32) forming the i^{th} radiation string (32i) before attaining the target volume (Vt), wherein a radiation module (32) is formed by a filter module (12) and a shifter module (22) aligned along the irradiation axis (Z),
• controlling the electromagnetic elements (4) for orienting the pencil beam (Bj) through a first radiation module (32) formed by a first filter module (12) of the i^{th} filter string (12i) and a first shifter module (22) of the i^{th} shifter string (22i), and towards a first spot (Sj) of an i^{th} scanning string formed by a sequence of spots (Sj) distributed along the X-axis, the spots being arranged in a plurality of scanning strings (I, i+1...) distributed along the Y-axis to define an array of spots arranged on a plane (X, Y) representative of a projection onto the plane (X, Y) of the treatment volume (Vt),
• after the pencil beam (Bj) traversing the first spot (Sj) of the i^{th} scanning string delivered a predefined dose (Dj), the electromagnetic elements (4) are controlled for sequentially orienting the pencil beams (Bj) of the i^{th} scanning string through the sequence of radiation modules (32) forming the the i^{th} radiation string (32i), and towards the sequence of spots (Sj) of the i^{th} scanning string.,
• for all filter modules (12) forming the i^{th} filter string (12i) and for all shifter modules (22) forming the i^{th} shifter string (22i), after the pencil beam (Bj) has traversed an x^{th} radiation module (32) and moves to a next (x+1)^{th} radiation module (32) along the X-axis, moving along the Y-axis the j^{th} filter row (10) and the x^{th} shifter row (20) to yield the radiation module (32) required for irradiating the x^{th} radiation module of the next (i + 1)^{th} radiation string (32(i+1)) required for irradiating a next (i+1)^{th} scanning string of spots (Sj) according to the predefined treatment.

12. Treatment station according to claims 10 and 11, wherein after the pencil beam (Bj) traversing a last spot (Sj) of the i^{th} scanning string delivered a predefined dose (Dj) into a corresponding last specific volume (Vj), the one or more processors are configured for,
• controlling the electromagnetic elements (4) or the translation system of the couch (5) as defined in claim 10 for orienting the pencil beam (Bj) through a first spot of an (i+ 1)^{th} scanning string adjacent along the Y-axis to the i^{th} scanning string, and
• repeating the steps of claim 11 for the spots on the (i+ 1)^{th} scanning string,
• repeating the foregoing steps for all of the plurality of scanning strings forming the array of spots (Sj) which have not yet received the dose according to the predefined treatment plan.

13. Treatment station according to claim 10, wherein the couch (5) is static and wherein, to ensure that the beam paths followed by the pencil beams (Bj) always cross a corresponding filter module (12) of the filter string (12i) and a corresponding shifter module (22) of the shifter string (22i), the one or more processors are configured for synchronizing the electromagnetic elements (4) and
• the translation system of the shaping device for ensuring that as the pencil beam is deviated along the Y-axis, the shaping device or the radiation string (32i) is also translated along the Y-axis, or
• a moving of the filter string (12i) and the shifter string (22i) along the Y-axis relative to the shaping device (30) which remains static relative to the target volume (Vt), in order to keep in alignment with the pencil beam along the Y-axis.

## Patentansprüche

1. Dynamisches Ridge-Filter (1) zum Formen einer Dosisabscheidungszone durch Strahlung mit Strahlen geladener Teilchen, vorzugsweise mit Protonen- oder anderen Leichtionenstrahlen, und zum Abscheiden von Dosen (Dj), die durch Bleistiftstrahlen (Bj) aufgebracht werden, die sich entlang entsprechender Bestrahlungsachsen (Zj) ausbreiten, die sich durch ein Bleistiftstrahlabtast(PBS)-Systems aus einer zentralen Bestrahlungsachse (Z) auffächern und in einer Sequenz an spezifische Volumina (Vj) entlang einer X-Achse abgegeben werden, um spezifische Volumenstreifen zu bilden, die nebeneinander entlang einer Y-Achse quer, vorzugsweise normal, zur X-Achse verteilt sind, wobei die spezifischen Volumina (Vj) in Kombination ein Zielvolumen (Vt) definieren, das die Tumorzellen umfasst,
wobei das dynamische Ridge-Filter eine Anzahl von Energiestreuungseinheiten (11) umfasst, wobei jede Energiestreuungseinheit (11) durch eine entsprechende Energiestreuungskapazität zum Streuen der Dosis gekennzeichnet ist, die entlang der Bestrahlungsachse (Zj) abgeschieden wird, indem die entsprechenden Bleistiftstrahlen (Bj) eine oder mehrere Energiestreuungseinheiten (11) durchqueren,
**dadurch gekennzeichnet, dass**
• die Energiestreuungseinheiten (11) in Filtermodulen (12) verteilt sind, wobei jedes Filtermodul (12) eine oder mehrere Energiestreuungseinheiten (11) stützt und eine Fläche normal zur zentralen Bestrahlungsachse (Z) aufweist, die mit einem Querschnitt des entsprechenden Bleistiftstrahls (Bj) kompatibel ist, wobei die Filtermodule (12) in unterschiedlichen Filterreihen (10) angeordnet sind, die sich jeweils entlang der Reihenrichtung (Y) erstrecken,
• jede Filterreihe (10) eine Anzahl von ungleichen Filtermodulen (12) umfasst, wobei jedes Filtermodul einer Filterreihe (10) eine von den anderen Filtermodulen (12) derselben Filterreihe (10) unterschiedliche Energiestreuungskapazität aufweist, wobei
∘ jede Filterreihe unabhängig verschoben werden kann, um einen Filterstrang (12i) entlang der X-Achse zu bilden, und
∘ der Filterstrang dazu ausgelegt ist, die Dosisabscheidung in den spezifischen Volumina (Vj) zu formen, die den spezifischen Volumenstreifen bilden, der dem Filterstrang entlang der Bestrahlungsachsen (Zj) zugewandt ist.

2. Dynamisches Ridge-Filter (1) nach Anspruch 1, wobei die Energiestreuungseinheiten (11) in Form von Spitzen mit generalisierter zylindrischer, vorzugsweise prismatischer Geometrie oder mit kegelförmiger Geometrie, abgestumpft oder nicht, vorzugsweise pyramidenförmiger Geometrie vorliegen, die auf einer Basis der Filtermodule (12) abgestützt und im Gebrauch so ausgelegt sind, dass sich eine Hauptachse der Spitzen parallel zu den entsprechenden Bleistiftstrahlachsen (Zj) erstreckt.

3. Dynamisches Ridge-Filter (1) nach Anspruch 1, wobei die Energiestreuungseinheiten (11) in Form von Mündungen mit generalisierter zylindrischer oder kegelförmiger Geometrie vorliegen, die in eine Stützbasis der Filtermodule (12) eindringen, wobei sich jede Mündung von einer Blendenöffnung an einer Oberfläche der Stützbasis erstreckt und bis zu einer gegebenen Tiefe eindringt, wodurch eine resultierende Materialdicke der Stützbasis verbleibt, und im Gebrauch so ausgelegt sind, dass sich eine Hauptachse der Hohlräume parallel zu den entsprechenden Bleistiftstrahlachsen (Zj) erstreckt.

4. Dynamisches Ridge-Filter (1) nach einem der vorhergehenden Ansprüche, wobei jedes Filtermodul (12) eine oder mehrere Energiestreuungseinheiten (11) umfasst, die jeweils durch mehrere Streuungsuntereinheiten (11a-11c) mit unterschiedlichen Querschnittsflächen (Ai) normal zur Bestrahlungsachse (Zj) gebildet sind und entlang einer Hauptachse der Energiestreuungseinheit (11) übereinander gestapelt sind, die im Gebrauch so ausgelegt ist, dass sie sich parallel zur Bestrahlungsachse (Zj) erstreckt, wobei alle Streuungsuntereinheiten (11a-11c) des Stapels unterschiedliche Energiestreuungskapazitäten aufweisen.

5. Dynamisches Ridge-Filter (1) nach einem der vorhergehenden Ansprüche, wobei alle Filterreihen (10) identisch sind und eine gleiche Auswahl von Filtermodulen (12) umfassen.

6. Dynamische Reichweitenverstellvorrichtung (2) zum Formen einer Dosisabscheidungszone durch Strahlung mit Strahlen geladener Teilchen, vorzugsweise mit Protonen- oder anderen Leichtionenstrahlen, und zum Abscheiden von Dosen (Dj), die durch Bleistiftstrahlen (Bj) aufgebracht werden, die sich entlang entsprechender Bestrahlungsachsen (Zj) ausbreiten, die sich durch ein Bleistiftstrahlabtast(PBS)-Systems aus einer zentralen Bestrahlungsachse (Z) auffächern und in einer Sequenz an spezifische Volumina (Vj) entlang einer X-Achse abgegeben werden, um spezifische Volumenstreifen zu bilden, die nebeneinander entlang einer Y-Achse quer, vorzugsweise normal, zur X-Achse verteilt sind, wobei die spezifischen Volumina (Vj) in Kombination ein Zielvolumen (Vt) definieren, das die Tumorzellen umfasst,
wobei die Reichweitenverstellvorrichtung (2) eine Materialdicke aufweist, die über eine Fläche der Reichweitenverstellvorrichtung (2) variiert, wobei die Fläche so ausgelegt ist, dass sie im Gebrauch im Wesentlichen normal zur Bestrahlungsachse (Zj) ist, wobei ein Wert der Dicke eine entsprechende Menge an absorbierter Energie (ΔE) des Bleistiftstrahls (Bj) der geladenen Teilchen bestimmt, die die Dicke durchqueren, **dadurch gekennzeichnet, dass**
• die Reichweitenverstellvorrichtung Verstellvorrichtungsmodule (22) umfasst, die nebeneinander angeordnet sind, um Verstellvorrichtungsreihen (20) zu bilden, wobei jede Verstellvorrichtungsreihe (20) eine Auswahl von Verstellvorrichtungsmodulen (22) umfasst, die eine konstante oder nahezu konstante Dicke aufweisen, die sich von den anderen Verstellvorrichtungsmodulen (22) derselben Verstellvorrichtungsreihe (20) unterscheidet, und dazu ausgelegt sind, unterschiedliche Mengen an absorbierter Referenzenergie (ΔE) von dem Bleistiftstrahl (Bj) zu absorbieren, wobei
• jede Verstellvorrichtungsreihe (20) unabhängig verschoben werden kann, um einem Verstellvorrichtungsstrang (22i) entlang der X-Achse zu bilden,
• der Verstellvorrichtungsstrang dazu ausgelegt ist, entlang der Bestrahlungsachsen (Zj) die Dosisabscheidung in den spezifischen Volumina (Vj) zu formen, die den spezifischen Volumenstreifen bilden, der dem Verstellvorrichtungsstrang entlang der Bestrahlungsachsen (Zj) zugewandt ist.

7. Dynamische Reichweitenverstellvorrichtung (2) nach Anspruch 6, wobei alle Verstellvorrichtungsreihen (20) identisch sind und eine gleiche Auswahl von Verstellvorrichtungsmodulen (22) umfassen.

8. Dynamische Formungsvorrichtung (30) zum Formen einer Dosisabscheidungszone durch Strahlung mit Strahlen geladener Teilchen, vorzugsweise mit Protonenstrahlen, und zum Abscheiden von Dosen (Dj), die durch Bleistiftstrahlen (Bj) aufgebracht werden, die sich entlang entsprechender Bestrahlungsachsen (Zj) ausbreiten, die sich mit einem Bleistiftstrahlabtast(PBS)-Systems aus einer zentralen Bestrahlungsachse (Z) auffächern und in eine Sequenz von spezifischen Volumina (Vj) entlang einer X-Achse abgegeben werden, um spezifische Volumenstreifen zu bilden, die nebeneinander entlang einer Y-Achse quer, vorzugsweise normal, zur X-Achse verteilt sind, wobei die spezifischen Volumina (Vj) in Kombination ein Zielvolumen (Vt) definieren, das die Tumorzellen umfasst,
**dadurch gekennzeichnet, dass** sie mindestens ein dynamisches Ridge-Filter (1) nach einem der Ansprüche 1 bis 5 und mindestens eine dynamische Reichweitenverstellvorrichtung (2) nach Anspruch 6 oder 7 umfasst. die in einer Sequenz entlang eines Strahlengangs eines Bleistiftstrahls (Bj) angeordnet sind, so dass der Filterstrang (12i) und der Verstellvorrichtungsstrang (22i) entlang einer Richtung, die im Gebrauch parallel zur Bestrahlungsachse (Zj) ist, einander zugewandt sind, um in Kombination einen Strahlungsstrang (32i) zu bilden.

9. Behandlungsstation zum Formen einer Dosisabscheidungszone durch Strahlung mit Strahlen geladener Teilchen, vorzugsweise mit Protonenstrahlen, und zum Abscheiden von Dosen (Dj), die durch Bleistiftstrahlen (Bj) aufgebracht werden, die sich entlang entsprechender Bestrahlungsachsen (Zj) ausbreiten, die sich mit einem Bleistiftstrahlabtast(PBS)-Systems aus einer zentralen Bestrahlungsachse (Z) auffächern und in eine Sequenz von spezifischen Volumina (Vj) entlang einer X-Achse abgegeben werden, um spezifische Volumenstreifen zu bilden, die nebeneinander entlang einer Y-Achse quer, vorzugsweise normal, zur X-Achse verteilt sind, wobei die spezifischen Volumina (Vj) in Kombination ein Zielvolumen (Vt) definieren, das die Tumorzellen umfasst, wobei die Behandlungsstation umfasst,
• eine Quelle von Bleistiftstrahlen (Bj) beschleunigter geladener Teilchen, vorzugsweise von Protonen,
• eine Düse (3) zum Richten der Bleistiftstrahlen (Bj) beschleunigter geladener Teilchen auf das Zielvolumen (Vt), wobei die Düse elektromagnetische Elemente (4) umfasst, die dazu ausgelegt sind, den Bleistiftstrahl (Bj) abzulenken, um entlang mindestens der X-Achse abzutasten, während die Düse statisch bleibt,
• ein Ridge-Filter,
• eine Reichweitenverstellvorrichtung,
• eine Liege oder einen Stuhl (5) zum Aufnehmen des Patienten in Rückenlage, Bauchlage, sitzender oder stehender Position,
• einen oder mehrere Prozessoren, die zum Steuern verschiedener Komponenten der Behandlungsstation ausgelegt sind,
**dadurch gekennzeichnet, dass**
• das Ridge-Filter ein dynamisches Ridge-Filter (1) nach einem der Ansprüche 1 bis 5 ist,
• die Reichweitenverstellvorrichtung eine dynamische Reichweitenverstellvorrichtung (2) nach Anspruch 6 oder 7 ist,
wobei das dynamische Ridge-Filter (1) und die dynamische Reichweitenverstellvorrichtung (2) so angeordnet sind, dass sie eine Formungsvorrichtung nach Anspruch 8 bilden.

10. Behandlungsstation nach Anspruch 9, wobei entweder,
• die elektromagnetischen Elemente (4) dazu ausgelegt sind, den Bleistiftstrahl (Bj) abzulenken, um auch entlang der Y-Achse abzutasten, während die Düse statisch bleibt, und
∘ die Formungsvorrichtung (30) mit einem Translationssystem versehen ist, das dazu ausgelegt ist, einer Translation des Bleistiftstrahls entlang der Y-Achse zu folgen, so dass sich der Filterstrang (12i) und der Verstellvorrichtungsstrang (22i) zusammen mit der Formungsvorrichtung (30) bewegen, wobei sie in Ausrichtung mit dem Bleistiftstrahl entlang der Y-Achse gehalten werden, oder
o sich der Filterstrang (12i) und der Verstellvorrichtungsstrang (22i) entlang der Y-Achse relativ zur Formungsvorrichtung (30) bewegen, die vorzugsweise relativ zum Zielvolumen (Vt) statisch bleibt, um in Ausrichtung mit dem Bleistiftstrahl entlang der Y-Achse gehalten zu werden, oder
• die Liege (5) mit einem Translationssystem versehen ist, das zum translatorischen Bewegen der Liege entlang der Y-Achse ausgelegt ist, um die spezifischen Volumina (Vj) mit den entsprechenden Bleistiftstrahlen (Bj) auszurichten, während die Formungsvorrichtung (30) und der Filterstrang und der Verstellvorrichtungsstrang vorzugsweise relativ zum Zielvolumen statisch bleiben,
oder
• eine Kombination der vorstehenden Optionen.

11. Behandlungsstation nach Anspruch 9 oder 10, wobei der eine oder die mehreren Prozessoren zu Folgendem ausgelegt sind:
• Bewegen der Filterreihen (10) und der Verstellvorrichtungsreihen (20) entlang der Y-Achse, um eine Sequenz von Filtermodulen (12), die einen i-ten Filterstrang (12i) bilden, und eines i-ten Verstellvorrichtungsstrangs (22i), der sich entlang der X-Achse erstreckt, zu erhalten, um einen i-ten Strahlungsstrang (32i) gemäß einem vordefinierten Behandlungsplan zu erhalten, so dass die Strahlengänge der Bleistiftstrahlen (Bj) eines i-ten Abtaststrangs entsprechende Strahlungsmodule (32) der Sequenz von Strahlungsmodulen (32), die den i-ten Strahlungsstrang (32i) bilden, durchlaufen, bevor das Zielvolumen (Vt) erreicht wird, wobei ein Strahlungsmodul (32) durch ein Filtermodul (12) und ein Verstellvorrichtungsmodul (22) gebildet wird, die entlang der Bestrahlungsachse (Z) ausgerichtet sind,
• Steuern der elektromagnetischen Elemente (4) zum Ausrichten des Bleistiftstrahls (Bj) durch ein erstes Strahlungsmodul (32), das durch ein erstes Filtermodul (12) des i-ten Filterstrangs (12i) und ein erstes Verstellvorrichtungsmodul (22) des i-ten Verstellvorrichtungsstrangs (22i) gebildet wird, und auf einen ersten Punkt (Sj) eines i-ten Abtaststrangs, der durch eine Sequenz von Punkten (Sj) gebildet wird, die entlang der X-Achse verteilt sind, wobei die Punkte in mehreren Abtaststrängen (I, i+1...) angeordnet sind, die entlang der X-Achse verteilt sind, um eine Anordnung von Punkten zu definieren, die auf einer Ebene (X, Y) angeordnet sind, die eine Projektion auf die Ebene (X, Y) des Behandlungsvolumens (Vt) darstellt,
• nachdem der Bleistiftstrahl (Bj), der den ersten Punkt (Sj) des i-ten Abtaststrangs durchquert, eine vordefinierte Dosis (Dj) abgegeben hat, werden die elektromagnetischen Elemente (4) zur sequentiellen Ausrichtung der Bleistiftstrahlen (Bj) des i-ten Abtaststrangs durch die Sequenz von Strahlungsmodulen (32), die den i-ten Strahlungsstrang (32i) bilden, und auf die Sequenz von Punkten (Sj) des i-ten Abtaststrangs gesteuert.,
• für alle Filtermodule (12), die den i-ten Filterstrang (12i) bilden, und für alle Verstellvorrichtungsmodule (22), die den i-ten Verstellvorrichtungsstrang (22i) bilden, nachdem der Bleistiftstrahl (Bj) ein x-tes Strahlungsmodul (32) durchlaufen hat und sich zu einem nächsten (x+1)-ten Strahlungsmodul (32) entlang der X-Achse bewegt, Bewegen der j-ten Filterreihe (10) und der x-ten Verstellvorrichtungsreihe (20) entlang der Y-Achse, um das Strahlungsmodul (32) zu erhalten, das zur Bestrahlung des x-ten Strahlungsmoduls des nächsten (i+1)-ten Strahlungsstrangs (32(i+1)) erforderlich ist, das zur Bestrahlung eines nächsten (i+1)-ten Abtaststrangs von Punkten (Sj) gemäß der vordefinierten Behandlung erforderlich ist.

12. Behandlungsstation nach den Ansprüchen 10 und 11, wobei, nachdem der Bleistiftstrahl (Bj), der einen letzten Punkt (Sj) des i-ten Abtaststrangs durchquert, eine vordefinierte Dosis (Dj) in ein entsprechendes letztes spezifisches Volumen (Vj) abgegeben hat, der eine oder die mehreren Prozessoren zu Folgendem ausgelegt sind,
• Steuern der elektromagnetischen Elemente (4) oder des Translationssystems der Liege (5) wie in Anspruch 10 definiert zum Ausrichten des Bleistiftstrahls (Bj) durch einen ersten Punkt eines (i+1)-ten Abtaststrangs, der entlang der Y-Achse an den i-ten Abtaststrang angrenzt, und
• Wiederholen der Schritte nach Anspruch 11 für die Punkte auf dem (i+1)-ten Abtaststrang,
• Wiederholen der vorstehenden Schritte für alle der mehreren Abtaststränge, die die Anordnung von Punkten (Sj) bilden, die die Dosis gemäß dem vordefinierten Behandlungsplan noch nicht empfangen haben.

13. Behandlungsstation nach Anspruch 10, wobei die Liege (5) statisch ist und wobei, um sicherzustellen, dass die Strahlengänge, denen die Bleistiftstrahlen (Bj) folgen, immer ein entsprechendes Filtermodul (12) des Filterstrangs (12i) und ein entsprechendes Verstellvorrichtungsmodul (22) des Verstellvorrichtungsstrangs (22i) kreuzen, der eine oder die mehreren Prozessoren zum Synchronisieren der elektromagnetischen Elemente (4) ausgelegt sind und
• das Translationssystem der Formungsvorrichtung zum Sicherstellen, dass, während der Bleistiftstrahl entlang der Y-Achse abgelenkt wird, die Formungsvorrichtung oder der Strahlungsstrang (32i) auch entlang der Y-Achse translatorisch bewegt wird, oder
• ein Bewegen des Filterstrangs (12i) und des Verstellvorrichtungsstrangs (22i) entlang der Y-Achse relativ zur Formungsvorrichtung (30), die relativ zum Zielvolumen (Vt) statisch bleibt, um entlang der Y-Achse in Ausrichtung mit dem Bleistiftstrahl gehalten zu werden.

## Revendications

1. Filtre de crête (= « ridge filter ») dynamique (1) pour mettre en forme une zone de dépôt de dose par radiation avec des faisceaux de particules chargées, de préférence avec des faisceaux de protons ou d'autres faisceaux d'ions légers, et pour déposer des doses (Dj), appliquées par des faisceaux par pinceau (= « pencil beams ») (Bj) se propageant le long d'axes d'irradiation correspondants (Zj) se déployant autour d'un axe d'irradiation central (Z), par l'intermédiaire d'un système de balayage à faisceau par pinceau (« Pencil Beam Scanning », PBS), et déposées en séquence dans des volumes spécifiques (Vj) le long d'un axe X pour former des bandes de volume spécifique distribuées les unes à côté des autres le long d'un axe Y transversal, de préférence normal, à l'axe X, les volumes spécifiques (Vj) définissant en association un volume cible (Vt) comprenant les cellules tumorales,
dans lequel le filtre de crête dynamique comprend un nombre d'unités de dispersion d'énergie (11), chaque unité de dispersion d'énergie (11) étant **caractérisée par** une capacité de dispersion d'énergie correspondante pour disperser la dose déposée le long de l'axe d'irradiation (Zj) par les faisceaux par pinceau (= « pencil beams »)correspondants (Bj) traversant une ou plusieurs unités de dispersion d'énergie (11),
**caractérisé en ce que,**
• les unités de dispersion d'énergie (11) sont distribuées dans des modules de filtre (12), chaque module de filtre (12) supportant une ou plusieurs unités de dispersion d'énergie (11) et ayant une superficie normale à l'axe d'irradiation central (Z) compatible avec une section transversale du faisceau par pinceau (= « pencil beam »)correspondant (Bj), dans lequel les modules de filtre (12) sont agencés en différentes rangées de filtre (10), chacune s'étendant le long de la direction de rangée (Y),
• chaque rangée de filtre (10) comprend un nombre de différents modules de filtre (12), chaque module de filtre d'une rangée de filtre (10) ayant une capacité de dispersion d'énergie différente de celle des autres modules de filtre (12) de la même rangée de filtre (10), dans lequel
o chaque rangée de filtre peut être déplacée indépendamment pour former une chaîne de filtre (12i) le long de l'axe X, et
o la chaîne de filtre est configurée pour mettre en forme le dépôt de dose dans les volumes spécifiques (Vj) formant la bande de volume spécifique faisant face à la chaîne de filtre le long des axes d'irradiation (Zj).

2. Filtre de crête dynamique (1) selon la revendication 1, dans lequel les unités de dispersion d'énergie (11) sont sous la forme de pointes de géométrie cylindrique généralisée, de préférence prismatique, ou de géométrie conique, tronquée ou non, de préférence une géométrie pyramidale supportée sur une base des modules de filtre (12) et configurée en utilisation pour avoir un grand axe des pointes s'étendant parallèlement aux axes de faisceau par pinceau (= « pencil beam »)correspondants (Zj).

3. Filtre de crête dynamique (1) selon la revendication 1, dans lequel les unités de dispersion d'énergie (11) sont sous la forme d'orifices de géométrie cylindrique généralisée ou conique pénétrant dans une base de support des modules de filtre (12), chaque orifice s'étendant depuis un orifice d'ouverture à une surface de la base de support et pénétrant jusqu'à une profondeur donnée, laissant une épaisseur résultante de matériau de la base de support, et configuré en utilisation pour avoir un grand axe des cavités s'étendant parallèlement au faisceau par pinceau (= « pencil beam »))correspondant axes (Zj).

4. Filtre de crête dynamique (1) selon l'une quelconque des revendications précédentes, dans lequel chaque module de filtre (12) comprend une ou plusieurs unités de dispersion d'énergie (11) chacune formée par une pluralité de sous-unités de dispersion (11a-11c) ayant différentes superficies de section transversale (Ai) normales à l'axe d'irradiation (Zj), et empilées les unes sur les autres le long d'un grand axe de l'unité de dispersion d'énergie (11) configuré en utilisation pour s'étendre parallèlement à l'axe d'irradiation (Zj), dans lequel toutes les sous-unités de dispersion (11a-11c) de l'empilage ont différentes capacités de dispersion d'énergie.

5. Filtre de crête dynamique (1) selon l'une quelconque des revendications précédentes, dans lequel toutes les rangées de filtre (10) sont identiques et comprennent une même sélection de modules de filtre (12).

6. Organe de décalage de portée (= « range shifter ») dynamique (2) pour mettre en forme une zone de dépôt de dose par radiation avec des faisceaux de particules chargées, de préférence avec des faisceaux de protons ou d'autres faisceaux d'ions légers, et pour déposer des doses (Dj), appliquées par des faisceaux par pinceau (= « pencil beams ») (Bj) se propageant le long d'axes d'irradiation correspondants (Zj) se déployant autour d'un axe d'irradiation central (Z), par l'intermédiaire d'un système de balayage à faisceau par pinceau (« Pencil Beam Scanning », PBS) et fournies en séquence à des volumes spécifiques (Vj) le long d'un axe X pour former des bandes de volume spécifique distribuées les unes à côté des autres le long d'un axe Y transversal, de préférence normal, à l'axe X, les volumes spécifiques (Vj) définissant en association un volume cible (Vt) comprenant les cellules tumorales,
dans lequel l'organe de décalage de portée (2) a une épaisseur de matériau variant par-dessus une superficie de l'organe de décalage de portée (2), la superficie étant configurée pour être sensiblement normale à l'axe d'irradiation (Zj) en utilisation, dans lequel une valeur de l'épaisseur détermine une quantité correspondante d'énergie absorbée (ΔE) du faisceau par pinceau (= « pencil beam »))(Bj) des particules chargées traversant l'épaisseur,
**caractérisé en ce que,**
• l'organe de décalage de portée comprend des modules d'organe de décalage (22) agencés côte-à-côte pour former des rangées d'organe de décalage (20), dans lequel chaque rangée d'organe de décalage (20) comprend une sélection de modules d'organe de décalage (22) ayant une épaisseur constante ou presque constante différente des autres modules d'organe de décalage (22) de la même rangée d'organe de décalage (20), et configurés pour absorber différentes quantités d'énergie absorbée de référence (ΔE) du faisceau par pinceau (= « pencil beam »))(Bj), dans lequel
• chaque rangée d'organe de décalage (20) peut être déplacée indépendamment pour former une chaîne d'organe de décalage (22i) le long de l'axe X,
• la chaîne d'organe de décalage est configurée pour mettre en forme, le long des axes d'irradiation (Zj), le dépôt de dose dans les volumes spécifiques (Vj) formant la bande de volume spécifique faisant face à la chaîne d'organe de décalage le long des axes d'irradiation (Zj).

7. Organe de décalage de portée dynamique (2) selon la revendication 6, dans lequel toutes les rangées d'organe de décalage (20) sont identiques et comprennent une même sélection de modules d'organe de décalage (22).

8. Dispositif de mise en forme dynamique (30) pour mettre en forme une zone de dépôt de dose par radiation avec des faisceaux de particules chargées, de préférence avec des faisceaux de protons, et pour déposer des doses (Dj), appliquées par des faisceaux par pinceau (= « pencil beams ») (Bj) se propageant le long d'axes d'irradiation correspondants (Zj) se déployant autour d'un axe d'irradiation central (Z), avec un système de balayage à faisceau par pinceau (« Pencil Beam Scanning », PBS), et fournies en une séquence de volumes spécifiques (Vj) le long d'un axe X pour former des bandes de volume spécifique distribuées les unes à côté des autres le long d'un axe Y transversal, de préférence normal, à l'axe X, les volumes spécifiques (Vj) définissant en association un volume cible (Vt) comprenant les cellules tumorales,
**caractérisé en ce qu'**il comprend au moins un filtre de crête dynamique (1) selon l'une quelconque de la revendication 1 à 5 et au moins un organe de décalage de portée dynamique (2) selon la revendication 6 ou 7, disposés en une séquence le long d'un trajet de faisceau d'un faisceau par pinceau (= « pencil beam »))(Bj), de manière telle que la chaîne de filtre (12i) et la chaîne d'organe de décalage (22i) font face l'une à l'autre le long d'une direction, qui en utilisation est parallèle à l'axe d'irradiation (Zj), pour former en association une chaîne de radiation (32i).

9. Station de traitement pour mettre en forme une zone de dépôt de dose par radiation avec des faisceaux de particules chargées, de préférence avec des faisceaux de protons, et pour déposer des doses (Dj), appliquées par des faisceaux par pinceau (= « pencil beam ») (Bj) se propageant le long d'axes d'irradiation correspondants (Zj) se déployant d'un axe d'irradiation central (Z), avec un système de balayage à faisceau par pinceau Pencil Beam Scanning », PBS), et fournies en une séquence de volumes spécifiques (Vj) le long d'un axe X pour former des bandes de volume spécifique distribuées les unes à côté des autres le long d'un axe Y transversal, de préférence normal, à l'axe X, les volumes spécifiques (Vj) définissant en association un volume cible (Vt) comprenant les cellules tumorales, dans laquelle la station de traitement comprend,
• une source de faisceaux par pinceau (= « pencil beams ») (Bj) de particules chargées accélérées, de préférence de protons,
• une tuyère (3) pour diriger les faisceaux par pinceau (= « pencil beams ») (Bj) de particules chargées accélérées vers le volume cible (Vt), la tuyère comprenant des éléments électromagnétiques (4) configurés pour dévier le faisceau par pinceau (= « pencil beam »))(Bj) pour effectuer le balayage le long d'au moins l'axe X alors que la tuyère reste statique,
• un filtre de crête,
• un organe de décalage de portée,
• un divan ou siège (5) pour recevoir le patient en position couchée, sur le ventre, assise, ou debout,
• une ou plusieurs processeurs configurés pour commander divers composants de la station de traitement,
**caractérisé en ce que,**
• le filtre de crête (= ridge filter ») est un filtre de crête dynamique (1) selon l'une quelconque de revendications 1 à 5,
• l'organe de décalage de portée (= « range shifter ») est un organe de décalage de portée dynamique (2) selon la revendication 6 ou 7,
dans laquelle le filtre de crête dynamique (1) et l'organe de décalage de portée dynamique (2) sont agencés de manière telle à former un dispositif de mise en forme selon la revendication 8.

10. Station de traitement selon la revendication 9, dans laquelle
• les éléments électromagnétiques (4) sont configurés pour dévier le faisceau par pinceau (= « pencil beam »))(Bj) pour effectuer le balayage également le long de l'axe Y alors que la tuyère reste statique, et
∘ le dispositif de mise en forme (30) est pourvu d'un système de translation configuré pour suivre une translation du faisceau par pinceau (= « pencil beam ») le long de l'axe Y de manière telle que la chaîne de filtre (12i) et la chaîne d'organe de décalage (22i) se déplacent conjointement avec le dispositif de mise en forme (30), se maintenant en alignement avec le faisceau par pinceau (= « pencil beam »)le long de l'axe Y, ou
∘ la chaîne de filtre (12i) et la chaîne d'organe de décalage (22i) se déplacent le long de l'axe Y relativement au dispositif de mise en forme (30) qui de préférence reste statique relativement au volume cible (Vt), afin de se maintenir en alignement avec le faisceau par pinceau (= « pencil beam »)le long de l'axe Y, ou
• le divan (5) est pourvu d'un système de translation configuré pour translater le divan le long de l'axe Y pour aligner les volumes spécifiques (Vj) avec les faisceaux par pinceau (= « pencil beams »)correspondants (Bj), alors que le dispositif de mise en forme (30) et la chaîne de filtre et chaîne d'organe de décalage restent de préférence statiques relativement au volume cible, ou
• une combinaison des options précédentes.

11. Station de traitement selon la revendication 9 ou 10, dans laquelle l'un ou les plusieurs processeurs sont configurés pour :
• déplacer les rangées de filtre (10) et les rangées d'organe de décalage (20) le long de l'axe Y pour produire une séquence de modules de filtre (12) formant une i^{ième} chaîne de filtre (12i) et d'une i^{ième} chaîne d'organe de décalage (22i) s'étendant le long de l'axe X pour produire une i^{ième} chaîne de radiation (32i) selon un plan de traitement prédéfini, de manière telle que les trajets de faisceau du faisceaux par pinceau (= « pencil beams ») (Bj) d'une i^{ième} chaîne de balayage traversent des modules de radiation correspondants (32) de la séquence de modules de radiation (32) formant la i^{ième} chaîne de radiation (32i) avant d'atteindre le volume cible (Vt), dans laquelle un module de radiation (32) est formé par un module de filtre (12) et un module d'organe de décalage (22) alignés le long de l'axe d'irradiation (Z),
• commander les éléments électromagnétiques (4) pour orienter le faisceau par pinceau (= « pencil beam ») (Bj) à travers un premier module de radiation (32) formé par un premier module de filtre (12) de la i^{ième} chaîne de filtre (12i) et un premier module d'organe de décalage (22) de la i^{ième} chaîne d'organe de décalage (22i), et vers un premier point (Sj) d'une i^{ième} chaîne de balayage formée par une séquence de points (Sj) distribués le long de l'axe X, les points étant agencés en une pluralité de chaînes de balayage (I, i+1...) distribuées le long de l'axe Y pour définir un réseau de points agencés sur un plan (X, Y) représentatif d'une projection sur le plan (X, Y) du volume de traitement (Vt),
• après que le faisceau par pinceau (= « pencil beam ») (Bj) traversant le premier point (Sj) de la i^{ième} chaîne de balayage a fourni une dose prédéfinie (Dj), les éléments électromagnétiques (4) sont commandés pour séquentiellement orienter le faisceaux par pinceau (= « pencil beams ») (Bj) de la i^{ième} chaîne de balayage à travers la séquence de modules de radiation (32) formant la i^{ième} chaîne de radiation (32i), et vers la séquence de points (Sj) de la i^{ième} chaîne de balayage,
• pour tous les modules de filtre (12) formant la i^{ième} chaîne de filtre (12i) et pour tous les modules d'organe de décalage (22) formant la i^{ième} chaîne d'organe de décalage (22i), après que le faisceau par pinceau (= « pencil beam ») (Bj) a traversé un x^{ième} module de radiation (32) et se déplace jusqu'à un (x+1)^{ième} module de radiation (32) suivant le long de l'axe X, déplacer, le long de l'axe Y, la j^{ième} rangée de filtre (10) et la x^{ième} rangée d'organe de décalage (20) pour produire le module de radiation (32) requis pour irradier le x^{ième} module de radiation de la (i+1)^{ième} chaîne de radiation suivante (32(i+1)) requise pour irradier une (i+1)^{ième} chaîne de balayage suivante de points (Sj) selon le traitement prédéfini.

12. Station de traitement selon revendications 10 et 11, dans laquelle, après que le faisceau par pinceau (= « pencil beam ») (Bj) traversant un dernier point (Sj) de la i^{ième} chaîne de balayage a fourni une dose prédéfinie (Dj) dans un dernier volume spécifique correspondant (Vj), l'un ou les plusieurs processeurs sont configurés pour,
• commander les éléments électromagnétiques (4) ou le système de translation du divan (5) tel que défini dans la revendication 10 pour orienter le faisceau par pinceau (= « pencil beam ») (Bj) à travers un premier point d'une (i+1)^{ième} chaîne de balayage adjacente, le long de l'axe Y, vers la i^{ième} chaîne de balayage, et
• la répétition des étapes de la revendication 11 pour les points sur la (i+1)^{ième} chaîne de balayage,
• la répétition des étapes précédentes pour la totalité de la pluralité de chaînes de balayage formant le réseau de points (Sj) qui n'ont pas encore reçu la dose selon le plan de traitement prédéfini.

13. Station de traitement selon la revendication 10, dans laquelle le divan (5) est statique et dans laquelle, pour garantir que les trajets de faisceau suivis par le faisceaux par pinceau (= « pencil beam ») (Bj) croisent toujours un module de filtre correspondant (12) de la chaîne de filtre (12i) et un module d'organe de décalage correspondant (22) de la chaîne d'organe de décalage (22i), l'un ou les plusieurs processeurs sont configurés pour synchroniser les éléments électromagnétiques (4), et
• le système de translation du dispositif de mise en forme pour garantir que, quand le faisceau par pinceau (= « pencil beam »))est dévié le long de l'axe Y, le dispositif de mise en forme ou la chaîne de radiation (32i) est également translaté(e) le long de l'axe Y, ou
• un déplacement de la chaîne de filtre (12i) et de la chaîne d'organe de décalage (22i) le long de l'axe Y relativement au dispositif de mise en forme (30) qui reste statique relativement au volume cible (Vt), afin de maintenir l'alignement avec le faisceau par pinceau (= « pencil beam ») le long de l'axe Y.
